# EUROPEAN PATENT APPLICATION

(11) **EP 3 106 174 A1**
(43) Date of publication of application: **21.12.2016**
(21) Application number: 15749159.8
(22) Date of filing: 10.02.2015
(51) Int. Cl.: A61K 38/20, A61P 31/16

(54) **IMMUNOTHERAPEUTIC COMPOSITION, THERAPEUTIC METHOD AND DIAGNOSTIC METHOD**

(30) Priority: 11.02.2014 US 201461938307 P
(71) Applicant: Wang, Bin, Beijing 100083 (CN); Jin, Jin, Beijing 100085 (CN); Xie, Xiaoping, Beijing 100085 (CN); He, Zhonghuai, Beijing 100085 (CN); Yu, Qingling, Beijing 100085 (CN); Beijing Advaccine Biotechnology Co. Ltd., Beijing 100085 (CN)
(72) Inventor: WANG, Bin, No.17, Qinghua East Road, Haidian District (CN); JIN, Jin, No.26, ShangDi Xinxi Road, Haidian District (CN); XIE, Xiaoping, No.26, ShangDi Xinxi Road, Haidian District (CN); HE, Zhonghuai, No.26, ShangDi Xinxi Road, Haidian District (CN); YU, Qingling, No.26, ShangDi Xinxi Road, Haidian District (CN)
(74) Representative: Leonard, Thomas Charles
(86) International application number: PCT/CN2015/072708
(87) International publication number: WO 2015/120792

(57) **Abstract**

Disclosed herein is a immunotherapeutic composition comprising IL-17. Also disclosed herein is a method of increasing an immune response in a subject in need thereof. The method may include administering the immunotherapeutic composition to the subject. The increased immune response may protect against an influenza virus. Further disclosed herein is a method of diagnosing influenza viral infection.

## Description

### TECHNICAL FIELD

The present invention relates to an immunotherapeutic composition, a method of treating and/or preventing influenza, and a method of diagnosing influenza viral infection.

### BACKGROUND

Vaccines are used to stimulate an immune response in an individual to provide protection against and/or treatment for a particular disease (e.g., flu). Each year, numerous individuals contract and/or die from infection with the influenza virus. In particular, young children, the elderly, individuals with compromised immune systems, and health care workers are susceptible to influenza viral infection and often require hospitalization, thereby creating both economic and social burdens. Three influenza strains, in particular, have been of concern in the last decade. The H1N1 influenza A virus subtype is a common human influenza virus that has resulted in pandemic illness in 1919 (avian Spanish flu that killed 50 to 100 million people worldwide) and a swine-origin H1N1 pandemic that resulted in over 17,000 deaths by the start of 2010. The H7N9 avian influenza A virus subtype is a new influenza A subtype that arose in China in 2013 and resulted in 20 deaths and 100 people being infected, which is unusually high rate for a new infection. The H5N1 avian influenza A virus subtype arose in 2006 spreading globally after first appearing in Asia. It is epizootic (an epidemic in nonhumans) and panzootic (affecting animals of many species, especially over a wide area), killing tens of millions of birds and spurring the culling of hundreds of millions of others to stem its spread. Eleven outbreaks of H5N1 were reported worldwide in June 2008 in five countries (China, Egypt, Indonesia, Pakistan and Vietnam) compared to 65 outbreaks in June 2006 and 55 in June 2007. In July 2013 the WHO announced a total of 630 confirmed human cases which resulted in the deaths of 375 people since 2003.

Accordingly, the influenza virus is highly variable and thus each year, manufacturers are required to develop a new vaccine directed to the influenza virus (i.e., influenza vaccine) to account for this variability and newly emerging strains of the influenza virus. In other words, influenza vaccine production is based upon a prediction of which influenza strain(s) will be most prevalent amongst the population in any given year. Presently, these influenza vaccines are often produced by growing the influenza virus in chicken eggs, and thus, production is limited by the supply of fertile chickens and eggs and how well the influenza virus grows in the chicken eggs. As such, it is difficult to accelerate influenza vaccine production in response to pandemics and to quickly switch to a particular vaccine to target a particular influenza strain(s) with the influenza vaccine.

Accordingly, a need remains in the art for the development of a safe and effective immunotherapeutic composition that is applicable to many different influenza type A subtypes thereby providing widespread protection and promoting survival year to year.

### SUMMARY

The present invention is directed to an immunotherapeutic composition comprising IL-17. IL-17 can be encoded by SEQ ID NO:10 or SEQ ID NO:11. IL-17 can be a peptide comprising the amino acid sequence as set forth in SEQ ID NO:12. IL-17 can be encoded by a nucleic acid and the immunotherapeutic composition can further comprise an IL-17 peptide.

The immunotherapeutic composition can further comprise one or more other antigens selected from the group consisting of amiloride, an influenza antigen, and an interleukin. The influenza antigen can be selected from the group consisting of H1 HA, H2 HA, H3 HA, H5 HA, BHA antigen, and any combination thereof. The interleukin can be selected from the group consisting of IL-23, IL-33, IL-21 and the combination thereof.

The immunotherapeutic composition can further comprise a pharmaceutically acceptable excipient. IL-17 can be a monomer of an IL-17 peptide. IL-17 can be a dimer of IL-17 peptides.

The present invention is also directed to a method for treating a subject in need thereof against influenza virus, the method comprising administering a immunotherapeutic composition comprising IL-17 to the subject, wherein the subject is thereby resistant against multiple influenza virus subtypes. IL-17 can be a peptide comprising the amino acid sequence set forth in SEQ ID NO:12. The immunotherapeutic composition can further comprise a nucleic acid encoding the IL-17 peptide. The immunotherapeutic composition can further comprise further one or more other antigens selected from the group consisting of amiloride, an influenza antigen, and an interleukin.

Administering the immunotherapeutic composition can include electroporation or injection. An immune response of the subject can be increased by at least about 4-fold. The immune response of the subject can be increased by at least about 2-fold. The influenza virus can be an influenza A virus. The influenza A virus can be subtype H5N1, H7N9, or H1N1. The influenza A virus can be subtype H5N1. The influenza A virus can be subtype H7N9. An increased immune response of the subject can provide at least about 70% survival of influenza virus. The increased immune response of the subject can provide at least about 100% survival of influenza virus.

The present invention is further directed to a method for diagnosing influenza viral infection in a subject in need thereof. The method can comprise providing a sample obtained from the subject and measuring a level of IL-17 in the sample. The method can also comprise comparing the measured level of IL-17 to a threshold level of IL-17 and determining the subject is infected with influenza virus if the measured level of IL-17 is higher than the threshold level of IL-17. IL-17 can be a peptide comprising the amino acid sequence set forth in SEQ ID NO:12.

The sample can be a blood sample, a serum sample, or a plasma sample. The method can further comprise administering a immunotherapeutic composition comprising IL-17 to the subject infected with the influenza virus. IL-17 can be a peptide comprising the amino acid sequence set forth in SEQ ID NO:12. The method can further comprise distinguishing between a mild influenza viral infection and a severe influenza viral infection. The influenza virus can be subtype H5N1, H1N1, or H7N9.

### BRIEF DESCRIPTION OF THE DRAWINGS

FIG. 1 shows (A) a graph plotting mouse group vs. IL-17A serum levels; (B) a graph plotting days post infection (dpi) vs. percent survival; and (C) a graph plotting mouse group vs. viral load in lung tissue.
FIG. 2 shows (A) a graph plotting mouse group vs. IL-17A serum levels; (B) a graph plotting days post infection (dpi) vs. percent survival; and (C) a graph plotting mouse group vs. viral load in lung tissue.
FIG. 3 shows (A) a graph plotting days post infection (dpi) vs. percent weight loss for mice infected with influenza A virus H7N9; (B) a graph plotting dpi vs. percent weight loss for mice pre-treated with 0.1 µg rIL-17A and infected with influenza A virus H7N9; and (C) a graph plotting dpi vs. percent weight loss for mice pre-treated with 0.5 µg rIL-17A and infected with influenza A virus H7N9.

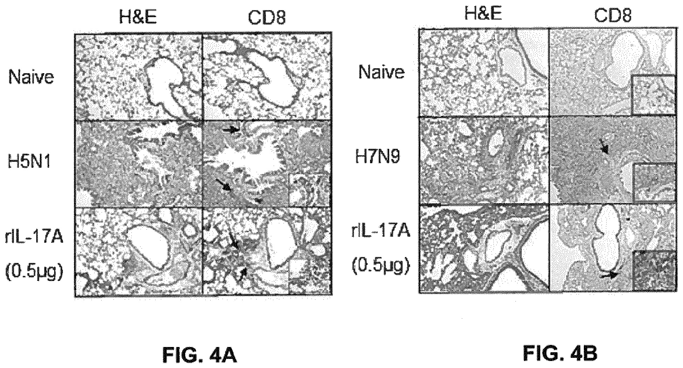

FIG. 4 shows hematoxylin and eosin (H&E) staining and immunohistochemistry with an antibody specific for CD8⁺ T cells (A) in lung tissue sections from naive mice (top panels), mice challenged with influenza A virus H5N1 (middle panels), and mice pre-treated with 0.5 µg recombinant IL-17A (rIL-17A) before challenge with influenza A virus H5N1 (bottom panels); and (B) in lung tissue sections from naive mice (top panels), mice challenged with influenza A virus H7N9 (middle panels), and mice pre-treated with 0.5 µg rIL-17A before challenge with influenza A virus H7N9 (bottom panels). The arrows indicate CD8⁺ T cells that have infiltrated the lung tissue in respective tissue sections.
FIG. 5 shows (A) a graph plotting human subject vs. IL-17A serum levels; (B) a graph plotting human subject vs. IL-17A serum levels; (C) a graph plotting human subject vs. IL-17A serum levels; and (D) a graph plotting human subject vs. IL-17A serum levels.
FIG. 6 shows (A) a graph plotting mouse group vs. cytokine level; (B) a graph plotting days post infection (dpi) vs. percent survival; and (C) a graph plotting dpi vs. percent survival.
FIG. 7 shows (A) a graph plotting mouse group vs. cytokine level; (B) a graph plotting mouse group vs. cytokine level; and (C) a graph plotting days post infection (dpi) vs. percent survival.
FIG. 8 shows (A) a graph plotting cell type vs. percent interferon-gamma (IFN-γ) producing cells; (B) a graph plotting days post infection (dpi) vs. percent survival; and (C) a graph plotting dpi vs. percent survival.
FIG. 9 shows (A) an illustration that depicts the experimental scheme for adoptive transfer of CD8⁺ T cells into recipient mice; (B) a graph plotting days post infection (dpi) vs. percent survival; and (C) a graph plotting dpi vs. percent survival.
FIG. 10 shows (A) a graph plotting mouse type vs. percent specific lysis; and (B) a graph plotting CD8⁺ T cell population vs. percent specific lysis.
FIG. 11 shows (A) the mRNA nucleotide sequence; (B) the coding nucleotide sequence; and (C) the amino acid sequence for *Mus musculus* (mouse) IL-17A.
FIG. 12 shows (A) the mRNA nucleotide sequence; (B) the coding nucleotide sequence; and (C) the amino acid sequence for *Homo sapiens* (human) IL-17A.
FIG. 13 shows (A) the optimized mouse IL-17A nucleotide sequence, in which the underlined sequence contains a BamHI site (GGA TCC) and a Kozak sequence (GCC ACC) and the double underlined sequence contains the stop codons TGA and TAA and a XhoI site (CTC GAG); (B) the optimized mouse IL-17A coding nucleotide sequence; and (C) the mouse IL-17A amino acid sequence encoded by the optimized nucleotide sequences of FIGS. 13A and 13B (i.e., SEQ ID NOS:7 and 8).
FIG. 14 shows (A) the optimized human IL-17A nucleotide sequence, in which the underlined sequence contains a BamHI site (GGA TCC) and a Kozak sequence (GCC ACC) and the double underlined sequence contains the stop codons TGA and TAA and a XhoI site (CTC GAG); (B) the optimized human IL-17A coding nucleotide sequence; and (C) the human IL-17A amino acid sequence encoded by the optimized nucleotide sequences of FIGS. 14A and 14B (i.e., SEQ ID NOS:10 and 11).

### DETAILED DESCRIPTION

The present invention relates to a immunotherapeutic composition comprising interleukin-17 (IL-17). The immunotherapeutic composition can be used to protect against and treat any number of influenza viruses, for example, pathogenic influenza A viruses H5N1, H1N1, and H7N9. Such protection and treatment can significantly increase the survival of a subject infected with influenza A viruses such as H5N1, H1N1 and H7N9. Survival is significantly increased because the immunotherapeutic composition increases the level of antiviral cytokine interferon-gamma produced by CD8⁺ T cells. These CD8⁺ T cells also have cytolytic activity, which destroy cells harboring the influenza A virus thereby preventing further production of influenza A virus particles that will lyse and exponentially infect other cells in the body. Accordingly, also provided herein is a method for treating and/or protecting against influenza viral infection by administering the immunotherapeutic composition to the subject in need thereof.

The level of IL-17 in serum increases upon infection with influenza virus. Accordingly, the present invention also relates to a method of diagnosing influenza viral infection based upon the serum level of IL-17 in the subject in need thereof.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

"Adjuvant" as used herein means any molecule added to the immunotherapeutic composition described herein to enhance the immunogenicity of the antigens.

"Coding sequence" or "encoding nucleic acid" as used herein means the nucleic acids (RNA or DNA molecule) that comprise a nucleotide sequence which encodes a protein. The coding sequence can further include initiation and termination signals operably linked to regulatory elements including a promoter and polyadenylation signal capable of directing expression in the cells of an individual or mammal to which the nucleic acid is administered.

"Complement" or "complementary" as used herein means a nucleic acid can mean Watson-Crick (e.g., A-T/U and C-G) or Hoogsteen base pairing between nucleotides or nucleotide analogs of nucleic acid molecules.

"Electroporation," "electro-permeabilization," or "electro-kinetic enhancement" ("EP") as used interchangeably herein means the use of a transmembrane electric field pulse to induce microscopic pathways (pores) in a bio-membrane; their presence allows biomolecules such as plasmids, oligonucleotides, siRNA, drugs, ions, and water to pass from one side of the cellular membrane to the other.

"Fragment" or "immunogenic fragment" as used herein means a nucleic acid sequence or a portion thereof that encodes a polypeptide capable of eliciting an immune response in a mammal. The fragments can be DNA fragments selected from at least one of the various nucleotide sequences that encode protein fragments set forth below. Fragments can comprise at least 10%, at least 20%, at least 30%, at least 40%, at least 50%, at least 60%, at least 70%, at least 80%, at least 90%, or at least 95% of one or more of the nucleic acid sequences set forth below. In some embodiments, fragments can comprise at least 20 nucleotides or more, at least 30 nucleotides or more, at least 40 nucleotides or more, at least 50 nucleotides or more, at least 60 nucleotides or more, at least 70 nucleotides or more, at least 80 nucleotides or more, at least 90 nucleotides or more, at least 100 nucleotides or more, at least 150 nucleotides or more, at least 200 nucleotides or more, at least 250 nucleotides or more, at least 300 nucleotides or more, at least 350 nucleotides or more, at least 400 nucleotides or more, at least 450 nucleotides or more, at least 500 nucleotides or more, at least 550 nucleotides or more, at least 600 nucleotides or more, at least 650 nucleotides or more, at least 700 nucleotides or more, at least 750 nucleotides or more, at least 800 nucleotides or more, at least 850 nucleotides or more, at least 900 nucleotides or more, at least 950 nucleotides or more, or at least 1000 nucleotides or more of at least one of the nucleic acid sequences set forth below.

Fragment or immunogenic fragment as used herein also means a polypeptide sequence or a portion thereof that is capable of eliciting an immune response in a mammal. The fragments can be polypeptide fragments selected from at least one of the various amino acid sequence set forth below. Fragments can comprise at least 10%, at least 20%, at least 30%, at least 40%, at least 50%, at least 60%, at least 70%, at least 80%, at least 90%, or at least 95% of one or more of the proteins set forth below. In some embodiments, fragments can comprise at least 20 amino acids or more, at least 30 amino acids or more, at least 40 amino acids or more, at least 50 amino acids or more, at least 60 amino acids or more, at least 70 amino acids or more, at least 80 amino acids or more, at least 90 amino acids or more, at least 100 amino acids or more, at least 110 amino acids or more, at least 120 amino acids or more, at least 130 amino acids or more, at least 140 amino acids or more, at least 150 amino acids or more, at least 160 amino acids or more, at least 170 amino acids or more, at least 180 amino acids or more, at least 190 amino acids or more, at least 200 amino acids or more, at least 210 amino acids or more, at least 220 amino acids or more, at least 230 amino acids or more, or at least 240 amino acids or more of at least one of the proteins set forth below.

"Genetic construct" as used herein refers to the DNA or RNA molecules that comprise a nucleotide sequence which encodes a protein. The coding sequence includes initiation and termination signals operably linked to regulatory elements including a promoter and polyadenylation signal capable of directing expression in the cells of the individual to whom the nucleic acid molecule is administered. As used herein, the term "expressible form" refers to gene constructs that contain the necessary regulatory elements operable linked to a coding sequence that encodes a protein such that when present in the cell of the individual, the coding sequence will be expressed.

"Identical" or "identity" as used herein in the context of two or more nucleic acids or polypeptide sequences, means that the sequences have a specified percentage of residues that are the same over a specified region. The percentage can be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of single sequence are included in the denominator but not the numerator of the calculation. When comparing DNA and RNA, thymine (T) and uracil (U) can be considered equivalent. Identity can be performed manually or by using a computer sequence algorithm such as BLAST or BLAST 2.0.

"Immune response" as used herein means the activation of a host's immune system, e.g., that of a mammal, in response to the introduction of antigen. The immune response can be in the form of a cellular or humoral response, or both.

"Nucleic acid" or "oligonucleotide" or "polynucleotide" as used herein means at least two nucleotides covalently linked together. The depiction of a single strand also defines the sequence of the complementary strand. Thus, a nucleic acid also encompasses the complementary strand of a depicted single strand. Many variants of a nucleic acid can be used for the same purpose as a given nucleic acid. Thus, a nucleic acid also encompasses substantially identical nucleic acids and complements thereof. A single strand provides a probe that can hybridize to a target sequence under stringent hybridization conditions. Thus, a nucleic acid also encompasses a probe that hybridizes under stringent hybridization conditions.

Nucleic acids can be single stranded or double stranded, or can contain portions of both double stranded and single stranded sequence. The nucleic acid can be DNA, both genomic and cDNA, RNA, or a hybrid, where the nucleic acid can contain combinations of deoxyribo- and ribo-nucleotides, and combinations of bases including uracil, adenine, thymine, cytosine, guanine, inosine, xanthine hypoxanthine, isocytosine and isoguanine. Nucleic acids can be obtained by chemical synthesis methods or by recombinant methods.

"Operably linked" as used herein means that expression of a gene is under the control of a promoter with which it is spatially connected. A promoter can be positioned 5' (upstream) or 3' (downstream) of a gene under its control. The distance between the promoter and a gene can be approximately the same as the distance between that promoter and the gene it controls in the gene from which the promoter is derived. As is known in the art, variation in this distance can be accommodated without loss of promoter function.

A "peptide," "protein," or "polypeptide" as used herein can mean a linked sequence of amino acids and can be natural, synthetic, or a modification or combination of natural and synthetic.

"Promoter" as used herein means a synthetic or naturally-derived molecule which is capable of conferring, activating or enhancing expression of a nucleic acid in a cell. A promoter can comprise one or more specific transcriptional regulatory sequences to further enhance expression and/or to alter the spatial expression and/or temporal expression of same. A promoter can also comprise distal enhancer or repressor elements, which can be located as much as several thousand base pairs from the start site of transcription. A promoter can be derived from sources including viral, bacterial, fungal, plants, insects, and animals. A promoter can regulate the expression of a gene component constitutively or differentially with respect to cell, the tissue or organ in which expression occurs or, with respect to the developmental stage at which expression occurs, or in response to external stimuli such as physiological stresses, pathogens, metal ions, or inducing agents. Representative examples of promoters include the bacteriophage T7 promoter, bacteriophage T3 promoter, SP6 promoter, lac operator-promoter, tac promoter, SV40 late promoter, SV40 early promoter, RSV-LTR promoter, CMV IE promoter, SV40 early promoter or SV40 late promoter and the CMV IE promoter.

"Signal peptide" and "leader sequence" are used interchangeably herein and refer to an amino acid sequence that can be linked at the amino terminus of a IL-17 protein (and/or one or more of the other antigens) set forth herein. Signal peptides/leader sequences typically direct localization of a protein. Signal peptides/leader sequences used herein preferably facilitate secretion of the protein from the cell in which it is produced. Signal peptides/leader sequences are often cleaved from the remainder of the protein, often referred to as the mature protein, upon secretion from the cell. Signal peptides/leader sequences are linked at the N terminus of the protein.

"Subject" as used herein can mean a mammal that wants to or is in need of being treated with the herein described immunotherapeutic composition. The mammal can be a human, chimpanzee, dog, cat, horse, cow, mouse, or rat.

"Substantially identical" as used herein can mean that a first and second amino acid sequence are at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%,or 99% over a region of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, 1100 or more amino acids. Substantially identical can also mean that a first nucleic acid sequence and a second nucleic acid sequence are at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%,or 99% over a region of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, 1100 or more nucleotides.

"Test sample" or "sample" as used herein generally refers to a biological material being tested for and/or suspected of containing an analyte of interest. The biological material may be derived from any biological source. Examples of biological materials include, but are not limited to, stool, whole blood, serum, plasma, red blood cells, platelets, interstitial fluid, salvia, ocular lens fluid, cerebral spinal fluid, sweat, urine, ascites fluid, mucous, nasal fluid, sputum, synovial fluid, peritoneal fluid, vaginal fluid, menses, amniotic fluid, semen, or soil, fermentation broths cell cultures, chemical reaction mixtures and the like. The test sample may be used directly as obtained from the biological source or following a pretreatment to modify the character of the sample. For example, such pretreatment may include preparing plasma from blood, diluting viscous fluids, and so forth. Methods of pretreatment may also involve filtration, precipitation, dilution, distillation, mixing, concentration, inactivation of interfering components, the addition of reagents, lysing, etc. If such methods of pretreatment are employed with respect to the test sample, such pretreatment methods are such that the analyte of interest remains in the test sample at a concentration proportional to that in an untreated test sample (e.g., namely, a test sample that is not subjected to any such pretreatment method(s)).

"Treatment" or "treating," as used herein can mean protecting of an animal from a disease through means of preventing, suppressing, repressing, or completely eliminating the disease. Preventing the disease involves administering a immunotherapeutic composition of the present invention to an animal prior to onset of the disease. Suppressing the disease involves administering a immunotherapeutic composition of the present invention to an animal after induction of the disease but before its clinical appearance. Repressing the disease involves administering a immunotherapeutic composition of the present invention to an animal after clinical appearance of the disease.

"Variant" used herein with respect to a nucleic acid means (i) a portion or fragment of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequences substantially identical thereto.

Variant can further be defined as a peptide or polypeptide that differs in amino acid sequence by the insertion, deletion, or conservative substitution of amino acids, but retain at least one biological activity. Representative examples of "biological activity" include the ability to be bound by a specific antibody or to promote an immune response. Variant can also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. Substitution of amino acids having similar hydrophilicity values can result in peptides retaining biological activity, for example immunogenicity, as is understood in the art. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

A variant may be a nucleic acid sequence that is substantially identical over the full length of the full gene sequence or a fragment thereof. The nucleic acid sequence may be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the gene sequence or a fragment thereof. A variant may be an amino acid sequence that is substantially identical over the full length of the amino acid sequence or fragment thereof. The amino acid sequence may be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the amino acid sequence or a fragment thereof.

"Vector" as used herein means a nucleic acid sequence containing an origin of replication. A vector can be a viral vector, bacteriophage, bacterial artificial chromosome or yeast artificial chromosome. A vector can be a DNA or RNA vector. A vector can be a self-replicating extrachromosomal vector, and preferably, is a DNA plasmid.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

### 2. Immunotherapeutic Composition

Provided herein is a immunotherapeutic composition comprising interleukin-17 (IL-17), a fragment thereof, a variant thereof, or a combination thereof. The immunotherapeutic composition can be used to protect against any number of influenza viruses thereby treating against a number of different flu based pathologies. Such protection and treatment can significantly increase the survival of (and prevention of death in) a subject infected with influenza virus as compared to a subject not administered the immunotherapeutic composition as described in more detail below.

The immunotherapeutic composition can significantly increase the immune response of the subject administered the immunotherapeutic composition as compared to an immune response of a subject not administered the immunotherapeutic composition, thereby protecting against and treating influenza viral infection. The immunotherapeutic composition can increase the CD8⁺ T cell immune response. The increased CD8⁺ T cell response can include an increased cytotoxic CD8⁺ T lymphocyte (CTL) response. The increased CTL response can include increasing in the subject administered the immunotherapeutic composition the amount of antigen-specific lysis of target cells. The increased CD8⁺ T cell response can include increasing in the subject administered the immunotherapeutic composition the population or frequency of CD8⁺ T cells secreting IFN-γ. The IFN-γ producing CD8⁺ T cells can have cytolytic activity of an infected cell thereby preventing new influenza A virus particles from being assembled by the cellular machinery and exponentially infecting other cells upon lysis of the infected cell.

The immunotherapeutic composition can increase the immune response of the subject administered the immunotherapeutic composition by about 0.5-fold to about 15-fold, about 0.5-fold to about 10-fold, or about 0.5-fold to about 8-fold as compared to the immune response of the subject not administered the immunotherapeutic composition. The immunotherapeutic composition can increase the immune response of the subject administered the immunotherapeutic composition by at least about 0.5-fold, at least about 1.0-fold, at least about 1.5-fold, at least about 2.0-fold, at least about 2.5-fold, at least about 3.0-fold, at least about 3.5-fold, at least about 4.0-fold, at least about 4.5-fold, at least about 5.0-fold, at least about 5.5-fold, at least about 6.0-fold, at least about 6.5-fold, at least about 7.0-fold, at least about 7.5-fold, at least about 8.0-fold, at least about 8.5-fold, at least about 9.0-fold, at least about 9.5-fold, at least about 10.0-fold, at least about 10.5-fold, at least about 11.0-fold, at least about 11.5-fold, at least about 12.0-fold, at least about 12.5-fold, at least about 13.0-fold, at least about 13.5-fold, at least about 14.0-fold, at least about 14.5-fold, or at least about 15.0-fold as compared to the immune response of the subject not administered the immunotherapeutic composition.

The immunotherapeutic composition can also increase the immune response of the subject administered the immunotherapeutic composition by about 50% to about 1500%, about 50% to about 1000%, or about 50% to about 800% as compared to the immune response of the subject not administered the immunotherapeutic composition. The immunotherapeutic composition can increase the immune response of the subject administered the immunotherapeutic composition by at least about 50%, at least about 100%, at least about 150%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, at least about 1000%, at least about 1050%, at least about 1100%, at least about 1150%, at least about 1200%, at least about 1250%, at least about 1300%, at least about 1350%, at least about 1450%, or at least about 1500% as compared to the immune response of the subject not administered the immunotherapeutic composition.

The immunotherapeutic composition can be a DNA immunotherapeutic composition, a peptide immunotherapeutic composition, or a combination DNA and peptide immunotherapeutic composition. The DNA immunotherapeutic composition can include a nucleic acid sequence encoding IL-17. The nucleic acid sequence can be DNA, RNA, cDNA, a variant thereof, a fragment thereof, or a combination thereof. The nucleic acid sequence can also include additional sequences that encode linker, leader, or tag sequences that are linked to IL-17 by a peptide bond. The peptide immunotherapeutic composition can include an IL-17 peptide, an IL-17 protein, a variant thereof, a fragment thereof, or a combination thereof. The combination DNA and peptide immunotherapeutic composition can include the above described nucleic acid sequence encoding IL-17 and the IL-17 peptide or protein, in which the IL-17 peptide or protein and the encoded IL-17 have the same amino acid sequence.

The immunotherapeutic composition can further include one or more other antigens, for example, but not limited to, a small molecule (e.g., amiloride), an influenza virus polynucleotide, an influenza virus peptide, a nucleic acid encoding a cytokine other than IL-17, and a cytokine peptide other than an IL-17 peptide. These one or more other antigens are described below. The one or more other antigens can be included in the above described DNA, peptide, or combination DNA and peptide immunotherapeutic compositions.

The immunotherapeutic composition of the present invention can have features required of effective immunotherapeutic compositions such as being safe so the immunotherapeutic composition itself does not cause illness or death; being protective against illness resulting from exposure to live pathogens such as viruses or bacteria; inducing neutralizing antibody to prevent infection of cells; inducing protective T cell against intracellular pathogens; and providing ease of administration, few side effects, biological stability, and low cost per dose.

The immunotherapeutic composition can further induce an immune response when administered to different tissues such as the muscle or the skin. The immunotherapeutic composition can further induce an immune response when administered via electroporation or injection or subcutaneously.

### a. IL-17

As described above, the immunotherapeutic composition comprises IL-17, a monomer thereof, a dimer thereof, a homodimer thereof, a heterodimer thereof, a fragment thereof, a variant thereof, or a combination thereof. The IL-17 may be IL-17A (also known as CTLA-8), IL-17B, IL-17C, IL-17D, IL-17E (also known as IL-25) and IL-17F. The IL-17 cytokines may be isolated and originate from multiple cell types, for example, T helper type 17 (Th17) cells, activated T cells, and epithelial cells. IL-17 cytokines have a highly conserved C-terminus, which contains a cysteine-knot fold structure, and are secreted as disulfide-linked dimers with the exception of IL-17B. IL-17B is secreted as a non-covalent dimer. Besides homodimers, heterodimers can be formed between IL-17A and IL-17F.

The immunotherapeutic composition can comprise about 0.001 µg to about 5 µg, about 0.005 µg to about 4 µg, about 0.01 µg to about 3 µg, about 0.05 µg to about 2 µg, or about 0.1 µg to about 1 µg of IL-17. The immunotherapeutic composition can comprise at least about 0.001 µg, at least about 0.002 µg, at least about 0.003 µg, at least about 0.004 µg, at least about 0.005 µg, at least about 0.006 µg, at least about 0.007 µg, at least about 0.008 µg, at least about 0.009 µg, at least about 0.01 µg, at least about 0.02 µg, at least about 0.03 µg, at least about 0.04 µg, at least about 0.05 µg, at least about 0.06 µg, at least about 0.07 µg, at least about 0.08 µg, at least about 0.09 µg, at least about 0.1 µg, at least about 0.2 µg, at least about 0.3 µg, at least about 0.4 µg, at least about 0.5 µg, at least about 0.6 µg, at least about 0.7 µg, at least about 0.8 µg, at least about 0.9 µg, at least about 1.0 µg, at least about 1.5 µg, at least about 2.0 µg, at least about 2.5 µg, at least about 3.0 µg, at least about 3.5 µg, at least about 4.0 µg, at least about 4.5 µg, or at least about 5.0 µg of IL-17.

The IL-17 cytokine of the immunotherapeutic composition signal through members of the IL-17 receptor family and activation of these receptors triggers intercellular pathways that induce the production of pro-inflammatory cytokines, for example, interferon-gamma (IFN-γ), IL-6, and tumor necrosis factor alpha (TNF-α). Accordingly, overexpression of the IL-17 cytokines can contribute to the development of autoimmune and inflammatory diseases such as asthma, rheumatoid arthritis, psoriasis, transplant rejection, inflammatory bowel disease, and multiple sclerosis.

IFN-γ has antiviral, immunoregulatory, and anti-tumor properties and can alter transcription in multiple genes to produce a variety of physiological and cellular responses. Some effects by IFN-γ include promoting natural killer (NK) cell activity, causing normal cells to increase expression of class I MHC molecules, increasing antigen presentation and lysosome activity in macrophages, inducing nitric oxide synthase (iNOS), and promoting Th1 differentiation to cellular immunity regarding cytotoxic CD8⁺ T cells while suppressing Th2 differentiation in humoral (antibody) response.

Cytotoxic CD8⁺ T cells (cytotoxic T lymphocytes (CTLs)) are a subgroup of T cells that induce the death of cells infected with viruses and other pathogens. Upon activation, CTLs undergo clonal expansion to produce effector cells that are antigen-specific. Effector CTLs release through a process of directed exocytosis (i.e., degranulation) molecules that kill infected or target cells, for example, perforin, granulysin, and granzyme. When no longer needed, many effector CTLs die, but some effector cells are retained as memory cells such that when the antigen is encountered again, the memory cells differentiate into effector cells to more quickly mount an immune response.

As described above, the immunotherapeutic composition can increase the immune response of the subject administered the immunotherapeutic composition as compared to the immune response of the subject not administered the immunotherapeutic composition. This increased immune response can include an increased CD8⁺ T cell response. The increased CD8⁺ T cell response can include an increased cytotoxic CD8⁺ T lymphocyte (CTL) response. The increased CTL response can include increasing in the subject administered the immunotherapeutic composition the amount of antigen-specific lysis of target cells. The increased CD8⁺ T cell response can include increasing in the subject administered the immunotherapeutic composition the population or frequency of CD8⁺ T cells secreting IFN-γ. The IFN-γ producing CD8⁺ T cells can have cytolytic activity.

In turn, the level of IFN-γ in the subject administered the immunotherapeutic composition can be increased by about 0.5-fold to about 15-fold, about 0.5-fold to about 10-fold, or about 0.5-fold to about 8-fold as compared to the level of IFN-γ in the subject not administered the immunotherapeutic composition. The level of IFN-γ in the subject administered the immunotherapeutic composition can be increased by at least about 0.5-fold, at least about 1.0-fold, at least about 1.5-fold, at least about 2.0-fold, at least about 2.5-fold, at least about 3.0-fold, at least about 3.5-fold, at least about 4.0-fold, at least about 4.5-fold, at least about 5.0-fold, at least about 5.5-fold, at least about 6.0-fold, at least about 6.5-fold, at least about 7.0-fold, at least about 7.5-fold, at least about 8.0-fold, at least about 8.5-fold, at least about 9.0-fold, at least about 9.5-fold, at least about 10.0-fold, at least about 10.5-fold, at least about 11.0-fold, at least about 11.5-fold, at least about 12.0-fold, at least about 12.5-fold, at least about 13.0-fold, at least about 13.5-fold, at least about 14.0-fold, at least about 14.5-fold, or at least about 15.0-fold as compared to the level of IFN-γ in the subject not administered the immunotherapeutic composition.

The level of IFN-γ in the subject administered the immunotherapeutic composition can also be increased by about 50% to about 1500%, about 50% to about 1000%, or about 50% to about 800% as compared to the level of IFN-γ in the subject not administered the immunotherapeutic composition. The level of IFN-γ in the subject administered the immunotherapeutic composition can also be increased by at least about 50%, at least about 100%, at least about 150%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, at least about 1000%, at least about 1050%, at least about 1100%, at least about 1150%, at least about 1200%, at least about 1250%, at least about 1300%, at least about 1350%, at least about 1450%, or at least about 1500% as compared to the level of IFN-γ in the subject not administered the immunotherapeutic composition.

### (1) IL-17A

IL-17 can be IL-17A, a fragment thereof, a variant thereof, or a combination thereof. IL-17A can be a monomer, a homodimer, or a heterodimer with IL-17F.

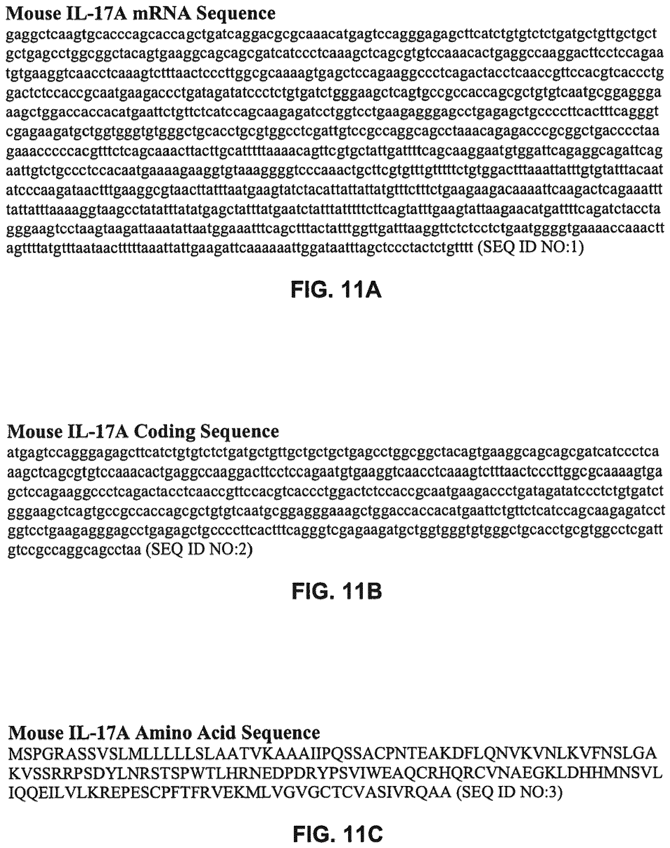

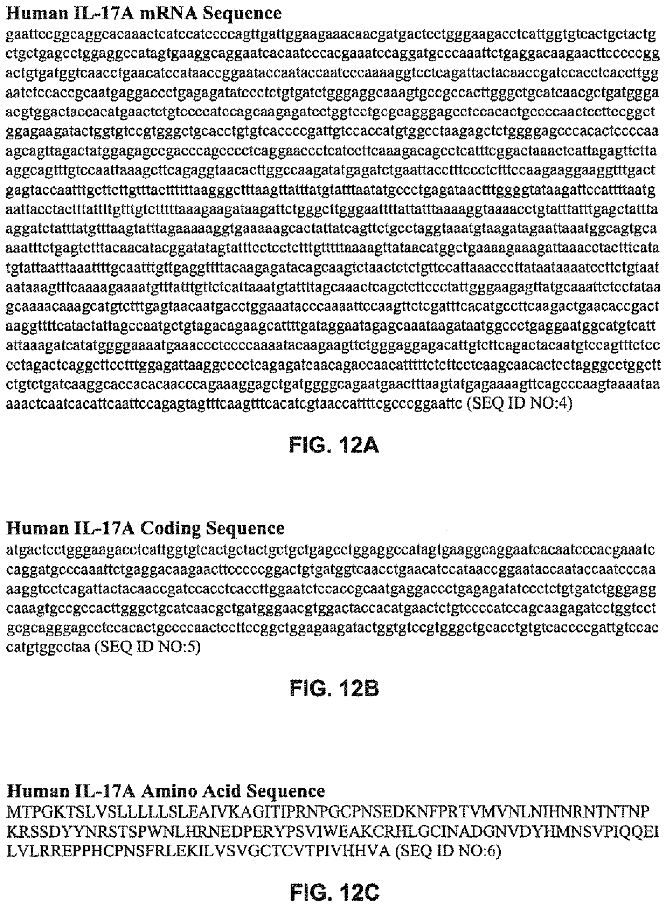

IL-17A can be an IL-17A protein from any number of organisms, for example, a mouse (*Mus musculus)* IL-17A protein and a human *(Homo sapiens)* IL-17A protein. The mouse IL-17A protein can have the amino acid sequence shown in FIG. 11C (i.e., SEQ ID NO:3), a fragment thereof, a variant thereof, or a combination thereof. The human IL-17A protein can have the amino acid sequence shown in FIG. 12C (i.e., SEQ ID NO:6), a fragment thereof, a variant thereof, or a combination thereof. The IL-17A protein may further comprise one or more additional amino acid sequence elements, for example, an immunoglobulin E (IgE) leader sequence (e.g., SEQ ID NO:14), an hemagglutinin (HA) tag, or both an IgE leader sequence and an HA tag.

A nucleic acid encoding IL-17A can be from any number of organisms, for example, mouse (*Mus musculus)* (FIGS. 11A and 11B, SEQ ID NOS:1 and 2, respectively) and human *(Homo sapiens*) (FIG. 12A and 12B, SEQ ID NOS:4 and 5, respectively). The nucleic acid encoding IL-17A can be optimized with regards to codon usage and corresponding RNA transcripts. The nucleic acid encoding IL-17A can be codon and RNA optimized for expression. The nucleic acid encoding IL-17A can include a Kozak sequence (e.g., GCC ACC) to increase the efficiency of translational initiation. The nucleic acid encoding IL-17A can include multiple stop codons (e.g., TGA TAA) to increase the efficiency of translational termination. The nucleic acid encoding IL-17A can also encode an immunoglobulin E (IgE) leader sequence. The IgE leader sequence can be located 5' to IL-17A in the nucleic acid. The nucleic acid encoding IL-17A can also include a nucleotide sequence encoding the IgE leader sequence (e.g., SEQ ID NO:13).

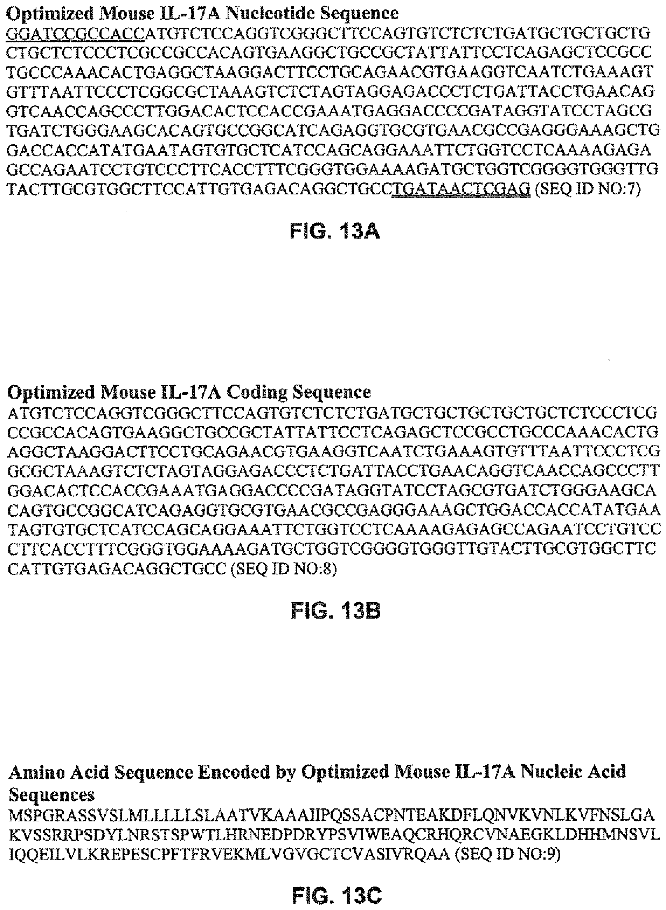

The optimized mouse IL-17A can be the nucleic acid sequence SEQ ID NO:7, which encodes SEQ ID NO:9 (FIGS. 13A and 13C). In some embodiments, the optimized mouse IL-17A can be the nucleic acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the nucleic acid sequence set forth in SEQ ID NO:7. In other embodiments, the optimized mouse IL-17A can be the nucleic acid sequence that encodes the amino acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the amino acid sequence set forth in SEQ ID NO:9.

The optimized mouse IL-17A can be the nucleic acid sequence SEQ ID NO:8, which encodes SEQ ID NO:9 (FIGS. 13B and 13C). In some embodiments, the optimized mouse IL-17A can be the nucleic acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the nucleic acid sequence set forth in SEQ ID NO:8.

The mouse IL-17A can be the amino acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the amino acid sequence set forth in SEQ ID NO:9.

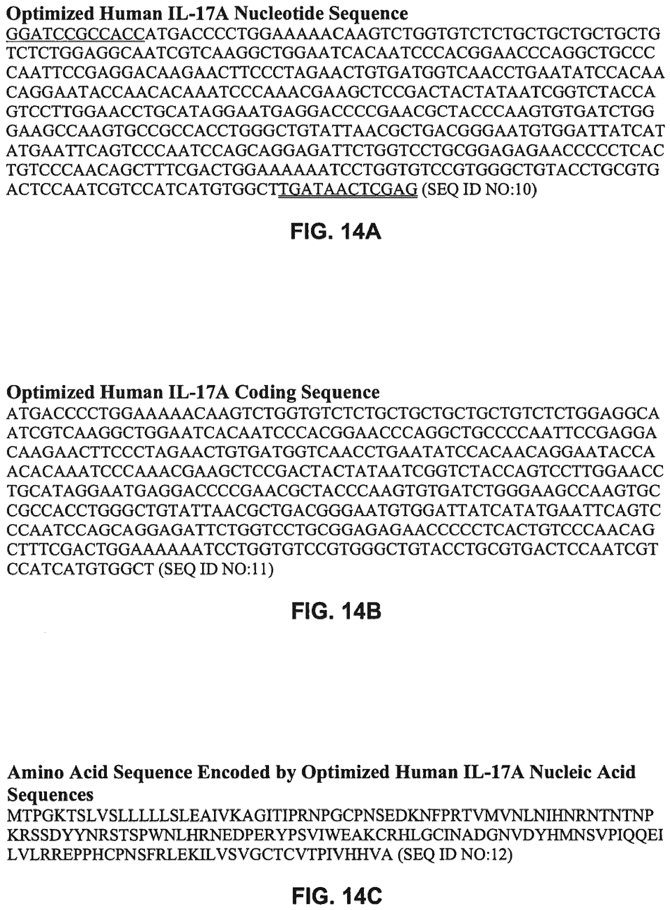

The optimized human IL-17A can be the nucleic acid sequence SEQ ID NO:10, which encodes SEQ ID NO:12 (FIGS. 14A and 14C). In some embodiments, the optimized human IL-17A can be the nucleic acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the nucleic acid sequence set forth in SEQ ID NO:10. In other embodiments, the optimized human IL-17A can be the nucleic acid sequence that encodes the amino acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the amino acid sequence set forth in SEQ ID NO:12.

The optimized human IL-17A can be the nucleic acid sequence SEQ ID NO:11, which encodes SEQ ID NO:12 (FIGS. 14B and 14C). In some embodiments, the optimized human IL-17A can be the nucleic acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the nucleic acid sequence set forth in SEQ ID NO:11.

The human IL-17A can be the amino acid sequence having at least about 80%, 81%, 82%, 83%, 84%, 85%, 86%, 87%, 88%, 89%, 90%, 91%, 92%, 93%, 94%, 95%, 96%, 97%, 98%, 99%, or 100% identity over an entire length of the amino acid sequence set forth in SEQ ID NO:12.

Immunogenic fragments of SEQ ID NO:9 and SEQ ID NO:12 can be provided. Immunogenic fragments can comprise at least 60%, at least 65%, at least 70%, at least 75%, at least 80%, at least 85%, at least 90%, at least 95%, at least 96%, at least 97%, at least 98%, or at least 99% of SEQ ID NO:9 and/or SEQ ID NO:12. In some embodiments, immunogenic fragments include a leader sequence, for example, an immunoglobulin leader sequence, such as the immunoglobulin E (IgE) leader sequence. In some embodiments, immunogenic fragments are free of a leader sequence.

Immunogenic fragments of proteins with amino acid sequences having identity to immunogenic fragments of SEQ ID NO:9 and 12 can be provided. Such fragments can comprise at least 60%, at least 65%, at least 70%, at least 75%, at least 75%, at least 80%, at least 85%, at least 90%, at least 95%, at least 96%, at least 97%, at least 98%, or at least 99% of proteins having 95% or greater identity to SEQ ID NO:9 and/or SEQ ID NO:12. Some embodiments relate to immunogenic fragments that have 96% or greater identity to the immunogenic fragments of IL-17A protein sequences herein. Some embodiments relate to immunogenic fragments that have 97% or greater identity to the immunogenic fragments of IL-17A protein sequences herein. Some embodiments relate to immunogenic fragments that have 98% or greater identity to the immunogenic fragments of IL-17A protein sequences herein. Some embodiments relate to immunogenic fragments that have 99% or greater identity to the immunogenic fragments of IL-17A protein sequences herein. In some embodiments, immunogenic fragments include a leader sequence, for example, an immunoglobulin leader sequence such as the IgE leader sequence. In some embodiments, the immunogenic fragments are free of a leader sequence.

Some embodiments relate to immunogenic fragments of SEQ ID NO:7, SEQ ID NO:8, SEQ ID NO:10 and SEQ ID NO:11. Immunogenic fragments can comprise at least 60%, at least 65%, at least 70%, at least 75%, at least 80%, at least 85%, at least 90%, at least 95%, at least 96%, at least 97%, at least 98%, or at least 99% of SEQ ID NO:7, SEQ ID NO:8, SEQ ID NO:10, and/or SEQ ID NO:11. In some embodiments, immunogenic fragments include sequences that encode a leader sequence, for example, an immunoglobulin leader sequence such as the IgE leader sequence. In some embodiments, immunogenic fragments are free of coding sequences that encode a leader sequence.

Immunogenic fragments of nucleic acids with nucleotide sequences having identity to immunogenic fragments of SEQ ID NO:7, SEQ ID NO:8, SEQ ID NO:10, and SEQ ID NO:11 can be provided. Such fragments can comprise at least 60%, at least 65%, at least 70%, at least 75%, at least 75%, at least 80%, at least 85%, at least 90%, at least 95%, at least 96%, at least 97%, at least 98%, or at least 99% of nucleic acids having 95% or greater identity to SEQ ID NO:7, SEQ ID NO:8, SEQ ID NO:10, and/or SEQ ID NO:11. Some embodiments relate to immunogenic fragments that have 96% or greater identity to the immunogenic fragments of IL-17A nucleic acid sequences herein. Some embodiments relate to immunogenic fragments that have 97% or greater identity to the immunogenic fragments of IL-17A nucleic acid sequences herein. Some embodiments relate to immunogenic fragments that have 98% or greater identity to the immunogenic fragments of IL-17A nucleic acid sequences herein. Some embodiments relate to immunogenic fragments that have 99% or greater identity to the immunogenic fragments of IL-17A nucleic sequences herein. In some embodiments, immunogenic fragments include sequences that encode a leader sequence, for example, an immunoglobulin leader sequence such as the IgE leader sequence. In some embodiments, immunogenic fragments are free of coding sequences that encode a leader sequence.

### b. Other Antigens

The immunotherapeutic composition can further comprise one or more other antigens in combination with IL-17. The antigen can be a small molecule, for example, amiloride or other influenza nucleic acid, protein or combination thereof as described below. The antigen can be a cytokine, for example, an interleukin other than IL-17 as described below.

The antigen can be a nucleic acid sequence, an amino acid sequence, or a combination thereof. The nucleic acid sequence can be DNA, RNA, cDNA, a variant thereof, a fragment thereof, or a combination thereof. The nucleic acid sequence can also include additional sequences that encode linker or tag sequences that are linked to the antigen by a peptide bond. The amino acid sequence can be a protein, a peptide, a variant thereof, a fragment thereof, or a combination thereof.

The antigen can be contained in a protein, a nucleic acid, or a fragment thereof, or a variant thereof, or a combination thereof from any number of organisms, for example, a virus, The antigen can be associated with influenza.

### (1) Amiloride

The immunotherapeutic composition may further comprise amiloride. Amiloride can have the following structure:

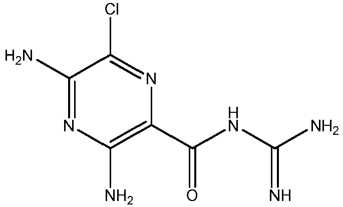

Amiloride can facilitate DNA uptake into a cell. Accordingly, the amiloride can be present in the immunotherapeutic composition in an amount that is capable of facilitating DNA uptake into a cell. Suitably effective increases in DNA uptake by a cell include by more than 5%, by more than 25%, or by more than 50%, as compared to the same immunotherapeutic composition composition without any amiloride.

Amiloride has several functional derivatives that are useful for facilitating DNA uptake into a cell. The functional derivatives can be formed by modifying the amino moieties either on the pyrimidine ring (at the 3-position) or the tri-amine. The functional derivatives of amiloride may be benzamil or 5-(N-ethyl-N-isopropyl) amiloride (EIPA).

### (2) Influenza Antigen

The immunotherapeutic composition may further comprise influenza antigen or fragment thereof, or variant thereof. The influenza antigens are those capable of eliciting an immune response in a mammal against one or more influenza serotypes. The antigen can comprise the full length translation product HA0, subunit HA1, subunit HA2, a variant thereof, a fragment thereof or a combination thereof. The influenza antigen comprise a full length or fragment of peptides isolated by any influenza A subtype such as H7N9, H1N1, H5N1 subtypes. The influenza hemagglutinin antigen can be a consensus sequence derived from multiple strains of influenza A serotype H1, a consensus sequence derived from multiple strains of influenza A serotype H2, a hybrid sequence containing portions of two different consensus sequences derived from different sets of multiple strains of influenza A serotype H1 or a consensus sequence derived from multiple strains of influenza B. The influenza hemagglutinin antigen can be from influenza B.

The influenza antigen can also contain at least one antigenic epitope that can be effective against particular influenza immunogens against which an immune response can be induced. The antigen may provide an entire repertoire of immunogenic sites and epitopes present in an intact influenza virus. The antigen may be a consensus hemagglutinin antigen sequence that can be derived from hemagglutinin antigen sequences from a plurality of influenza A virus strains of one serotype such as a plurality of influenza A virus strains of serotype H1 serotype H2, serotype H1N1, serotype H7N9, and/or serotype H7N1. The antigen may be a hybrid consensus hemagglutinin antigen sequence that can be derived from combining two different consensus hemagglutinin antigen sequences or portions thereof. Each of two different consensus hemagglutinin antigen sequences may be derived from a different set of a plurality of influenza A virus strains of one serotype such as a plurality of influenza A virus strains of serotype H1. The antigen may be a consensus hemagglutinin antigen sequence that can be derived from hemagglutinin antigen sequences from a plurality of influenza B virus strains.

In some embodiments, the influenza antigen can be H1 HA, H2 HA, H3 HA, H5 HA, or a BHA antigen. Alternatively, the influenza antigen can be a consensus hemagglutinin antigen comprising a consensus H1 amino acid sequence or a consensus H2 amino acid sequence. The consensus hemagglutinin antigen may be a synthetic hybrid consensus H1 sequence comprising portions of two different consensus H1 sequences, which are each derived from a different set of sequences from the other. An example of a consensus HA antigen that is a synthetic hybrid consensus H1 protein is a protein comprising the U2 amino acid sequence. The consensus hemagglutinin antigen may be a consensus hemagglutinin protein derived from hemagglutinin sequences from influenza B strains, such as a protein comprising the consensus BHA amino acid sequence.

The consensus hemagglutinin antigen may further comprise one or more additional amino acid sequence elements. The consensus hemagglutinin antigen may further comprise on its N-terminal an IgE or IgG leader amino acid sequence.The consensus hemagglutinin antigen may further comprise an immunogenic tag which is a unique immunogenic epitope that can be detected by readily available antibodies. An example of such an immunogenic tag is the 9 amino acid influenza HA Tag which may be linked on the consensus hemagglutinin C terminus. In some embodiments, consensus hemagglutinin antigen may further comprise on its N-terminal an IgE or IgG leader amino acid sequence and on its C terminal an HA tag.

The consensus hemagglutinin antigen may be a consensus hemagglutinin protein that consists of consensus influenza amino acid sequences or fragments and variants thereof. The consensus hemagglutinin antigen may be a consensus hemagglutinin protein that comprises non-influenza protein sequences and influenza protein sequences or fragments and variants thereof.

Examples of a consensus H1 protein include those that may consist of the consensus H1 amino acid sequence or those that further comprise additional elements such as an IgE leader sequence, or an HA Tag or both an IgE leader sequence and an HA Tag.

Examples of consensus H2 proteins include those that may consist of the consensus H2 amino acid sequence or those that further comprise an IgE leader sequence, or an HA Tag, or both an IgE leader sequence and an HA Tag.

Examples of hybrid consensus H1 proteins include those that may consist of the consensus U2 amino acid sequence or those that further comprise an IgE leader sequence, or an HA Tag, or both an IgE leader sequence and an HA Tag.

Examples of hybrid consensus influenza B hemagglutinin proteins include those that may consist of the consensus BHA amino acid sequence or it may comprise an IgE leader sequence, or an HA Tag, or both an IgE leader sequence and an HA Tag.

The consensus hemagglutinin protein can be encoded by a consensus hemagglutinin nucleic acid, a variant thereof or a fragment thereof. Unlike the consensus hemagglutinin protein which may be a consensus sequence derived from a plurality of different hemagglutinin sequences from different strains and variants, the consensus hemagglutinin nucleic acid refers to a nucleic acid sequence that encodes a consensus protein sequence and the coding sequences used may differ from those used to encode the particular amino acid sequences in the plurality of different hemagglutinin sequences from which the consensus hemagglutinin protein sequence is derived. The consensus nucleic acid sequence may be codon optimized and/or RNA optimized. The consensus hemagglutinin nucleic acid sequence may comprise a Kozak's sequence in the 5' untranslated region. The consensus hemagglutinin nucleic acid sequence may comprise nucleic acid sequences that encode a leader sequence. The coding sequence of an N terminal leader sequence is 5' of the hemagglutinin coding sequence. The N-terminal leader can facilitate secretion. The N-terminal leader can be an IgE leader or an IgG leader. The consensus hemagglutinin nucleic acid sequence can comprise nucleic acid sequences that encode an immunogenic tag. The immunogenic tag can be on the C terminus of the protein and the sequence encoding it is 3' of the HA coding sequence. The immunogenic tag provides a unique epitope for which there are readily available antibodies so that such antibodies can be used in assays to detect and confirm expression of the protein. The immunogenic tag can be an HA Tag at the C-terminus of the protein.

### (3) Interleukin

The immunotherapeutic composition may further comprise an interleukin or combination of other inteleukin and or cytokines other than IL-17. The interleukin can be IL-23, a variant thereof, a fragment thereof, or a combination thereof. The interleukin can also be IL-33, a variant thereof, a fragment thereof, or a combination thereof. The interleukin can be IL-21, a variant thereof, a fragment thereof, or a combination thereof.

### c. Vector

The immunotherapeutic composition can comprise one or more vectors that include a nucleic acid encoding IL-17 and may further comprise one or more antigens. The one or more vectors can be capable of expressing the IL-17 and may further comprise one or more antigens. The vector can have a nucleic acid sequence containing an origin of replication. The vector can be a plasmid, bacteriophage, bacterial artificial chromosome or yeast artificial chromosome. The vector can be either a self-replication extra chromosomal vector, or a vector which integrates into a host genome.

The one or more vectors can be an expression construct, which is generally a plasmid that is used to introduce a specific gene into a target cell. Once the expression vector is inside the cell, the protein that is encoded by the gene is produced by the cellular-transcription and translation machinery ribosomal complexes. The plasmid is frequently engineered to contain regulatory sequences that act as enhancer and promoter regions and lead to efficient transcription of the gene carried on the expression vector. The vectors of the present invention express large amounts of stable messenger RNA, and therefore proteins.

The vectors may have expression signals such as a strong promoter, a strong termination codon, adjustment of the distance between the promoter and the cloned gene, and the insertion of a transcription termination sequence and a PTIS (portable translation initiation sequence).

### (1) Expression Vector

The vector can be a circular plasmid or a linear nucleic acid. The circular plasmid and linear nucleic acid are capable of directing expression of a particular nucleotide sequence in an appropriate subject cell. The vector can have a promoter operably linked to the antigen-encoding nucleotide sequence, which may be operably linked to termination signals. The vector can also contain sequences required for proper translation of the nucleotide sequence. The vector comprising the nucleotide sequence of interest may be chimeric, meaning that at least one of its components is heterologous with respect to at least one of its other components. The expression of the nucleotide sequence in the expression cassette may be under the control of a constitutive promoter or of an inducible promoter, which initiates transcription only when the host cell is exposed to some particular external stimulus. In the case of a multicellular organism, the promoter can also be specific to a particular tissue or organ or stage of development.

### (2) Circular and Linear Vectors

The vector may be circular plasmid, which may transform a target cell by integration into the cellular genome or exist extrachromosomally (e.g., autonomous replicating plasmid with an origin of replication).

The vector can be pVAX, pcDNA3.0, or provax, or any other expression vector capable of expressing DNA encoding the antigen and enabling a cell to translate the sequence to an antigen that is recognized by the immune system.

Also provided herein is a linear nucleic acid, or linear expression cassette ("LEC"), that is capable of being efficiently delivered to a subject via electroporation and expressing one or more desired proteins, peptides and/or antigens. The LEC may be any linear DNA devoid of any phosphate backbone. The DNA may encode IL-17 and may further comprise one or more antigens. The LEC may contain a promoter, an intron, a stop codon, and/or a polyadenylation signal. The expression of the IL-17 may be controlled by the promoter and the promoter may further control the expression of the one or more antigens. The LEC may not contain any antibiotic resistance genes and/or a phosphate backbone. The LEC may not contain other nucleic acid sequences unrelated to the desired IL-17 gene expression, and the LEC may further not contain other nucleic acid sequence unrelated to the one or more antigens.

The LEC may be derived from any plasmid capable of being linearized. The plasmid may be capable of expressing the antigen. The plasmid can be pNP (Puerto Rico/34) or pM2 (New Caledonia/99). The plasmid may be WLV009, pVAX, pcDNA3.0, or provax, or any other expression vector capable of expressing DNA encoding IL-17 and may further comprise one or more antigens and enabling a cell to translate the sequence to IL-17 and the one or more antigens that is recognized by the immune system.

The LEC can be pcrM2. The LEC can be pcrNP. pcrNP and pcrMR can be derived from pNP (Puerto Rico/34) and pM2 (New Caledonia/99), respectively.

### (3) Promoter, Intron, Stop Codon, and Polyadenylation Signal

The vector may have a promoter. A promoter may be any promoter that is capable of driving gene expression and regulating expression of the isolated nucleic acid. Such a promoter is a cis-acting sequence element required for transcription via a DNA dependent RNA polymerase, which transcribes the IL-17 sequence described herein and may further transcribe the one or more antigen sequences described herein. Selection of the promoter used to direct expression of a heterologous nucleic acid depends on the particular application. The promoter may be positioned about the same distance from the transcription start in the vector as it is from the transcription start site in its natural setting. However, variation in this distance may be accommodated without loss of promoter function.

The promoter may be operably linked to the nucleic acid sequence encoding the antigen and signals required for efficient polyadenylation of the transcript, ribosome binding sites, and translation termination. The promoter may be a CMV promoter, SV40 early promoter, SV40 later promoter, metallothionein promoter, murine mammary tumor virus promoter, Rous sarcoma virus promoter, polyhedrin promoter, or another promoter shown effective for expression in eukaryotic cells.

The vector may include an enhancer and an intron with functional splice donor and acceptor sites. The vector may contain a transcription termination region downstream of the structural gene to provide for efficient termination. The termination region may be obtained from the same gene as the promoter sequence or may be obtained from different genes.

### d. Excipients and other components of the Immunotherapeutic composition

The immunotherapeutic composition may further comprise a pharmaceutically acceptable excipient. The pharmaceutically acceptable excipient can be functional molecules such as vehicles, adjuvants, carriers, or diluents. The pharmaceutically acceptable excipient can be a transfection facilitating agent, which can include surface active agents, such as immune-stimulating complexes (ISCOMS), Freunds incomplete adjuvant, LPS analog including monophosphoryl lipid A, muramyl peptides, quinone analogs, vesicles such as squalene and squalene, hyaluronic acid, lipids, liposomes, calcium ions, viral proteins, polyanions, polycations, or nanoparticles, or other known transfection facilitating agents.

The transfection facilitating agent is a polyanion, polycation, including poly-L-glutamate (LGS), or lipid. The transfection facilitating agent is poly-L-glutamate, and the poly-L-glutamate may be present in the immunotherapeutic composition at a concentration less than 6 mg/ml. The transfection facilitating agent may also include surface active agents such as immune-stimulating complexes (ISCOMS), Freunds incomplete adjuvant, LPS analog including monophosphoryl lipid A, muramyl peptides, quinone analogs and vesicles such as squalene and squalene, and hyaluronic acid may also be used administered in conjunction with the genetic construct. The DNA plasmid immunotherapeutic compositions may also include a transfection facilitating agent such as lipids, liposomes, including lecithin liposomes or other liposomes known in the art, as a DNA-liposome mixture (see for example WO9324640), calcium ions, viral proteins, polyanions, polycations, or nanoparticles, or other known transfection facilitating agents. The transfection facilitating agent is a polyanion, polycation, including poly-L-glutamate (LGS), or lipid. Concentration of the transfection agent in the immunotherapeutic composition is less than 4 mg/ml, less than 2 mg/ml, less than 1 mg/ml, less than 0.750 mg/ml, less than 0.500 mg/ml, less than 0.250 mg/ml, less than 0.100 mg/ml, less than 0.050 mg/ml, or less than 0.010 mg/ml.

The pharmaceutically acceptable excipient can be an adjuvant. The adjuvant can be other genes that are expressed in an alternative plasmid or are delivered as proteins in combination with the plasmid above in the immunotherapeutic composition. The adjuvant may be selected from the group consisting of: α-interferon(IFN-α), β-interferon (IFN-β), γ-interferon, platelet derived growth factor (PDGF), TNFα, TNFβ, GM-CSF, epidermal growth factor (EGF), cutaneous T cell-attracting chemokine (CTACK), epithelial thymus-expressed chemokine (TECK), mucosae-associated epithelial chemokine (MEC), IL-12, IL-15, MHC, CD80, CD86 including IL-15 having the signal sequence deleted and optionally including the signal peptide from IgE. The adjuvant can be IL-12, IL-15, IL-28, CTACK, TECK, platelet derived growth factor (PDGF), TNFα, TNFβ, GM-CSF, epidermal growth factor (EGF), IL-1, IL-2, IL-4, IL-5, IL-6, IL-10, IL-12, IL-18, or a combination thereof.

Other genes that can be useful as adjuvants include those encoding: MCP-1, MIP-la, MIP-1p, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, G-CSF, IL-4, mutant forms of IL-18, CD40, CD40L, vascular growth factor, fibroblast growth factor, IL-7, IL-22, nerve growth factor, vascular endothelial growth factor, Fas, TNF receptor, Flt, Apo-1, p55, WSL-1, DR3, TRAMP, Apo-3, AIR, LARD, NGRF, DR4, DR5, KILLER, TRAIL-R2, TRICK2, DR6, Caspase ICE, Fos, c-jun, Sp-1, Ap-1, Ap-2, p38, p65Rel, MyD88, IRAK, TRAF6, IkB, Inactive NIK, SAP K, SAP-1, JNK, interferon response genes, NFkB, Bax, TRAIL, TRAILrec, TRAILrecDRC5, TRAIL-R3, TRAIL-R4, RANK, RANK LIGAND, Ox40, Ox40 LIGAND, NKG2D, MICA, MICB, NKG2A, NKG2B, NKG2C, NKG2E, NKG2F, TAP1, TAP2 and functional fragments thereof.

The immunotherapeutic composition may further comprise a genetic immunotherapeutic composition facilitator agent as described in U.S. Serial No. 021,579 filed April 1, 1994, which is fully incorporated by reference.

The immunotherapeutic composition can be formulated according to the mode of administration to be used. An injectable immunotherapeutic composition pharmaceutical composition can be sterile, pyrogen free and particulate free. An isotonic formulation or solution can be used. Additives for isotonicity can include sodium chloride, dextrose, mannitol, sorbitol, and lactose. The immunotherapeutic composition can comprise a vasoconstriction agent. The isotonic solutions can include phosphate buffered saline. Immunotherapeutic composition can further comprise stabilizers including gelatin and albumin. The stabilizers can allow the formulation to be stable at room or ambient temperature for extended periods of time, including LGS or polycations or polyanions.

### 3. Method of Diagnosis

The present invention is also directed to a method for diagnosing influenza viral infection in a subject in need thereof. The method can include providing a sample obtained from the subject. The sample may be a blood sample, a plasma sample, or a serum sample. The method can also include measuring a level of IL-17 in the sample and comparing the measured level of IL-17 to a threshold level of IL-17 associated with a normal, healthy subject. The normal, healthy subject can be associated with a level of IL-17 that is less than about 50 pg/mL. 50 pg/mL can be a threshold level for distinguishing between the normal, healthy subject and the subject infected with influenza virus, in which the normal, healthy subject can be associated with a level of IL-17 that is less than about 50 pg/mL and the subject infected with influenza virus can be associated with a level of IL-17 that is greater than about 50 pg/mL.

The method can further include determining that the subject is infected with the influenza virus if the measured level of IL-17 is higher than the threshold level of IL-17 associated with a normal, healthy subject. The normal, healthy subject can be associated with a level of IL-17 that is less than about 50 pg/mL. If the measured level of IL-17 is greater than about 50 pg/mL, then the subject is infected with the influenza virus. If the measured level of IL-17 is less than about 50 pg/mL, then the subject is not infected with the influenza virus. The subject infected with influenza virus can be treated by administration of the immunotherapeutic composition. The subject associated with a level of IL-17 that is greater than about 50 pg/mL can be treated by administration of the immunotherapeutic composition.

The method may further comprise distinguishing between a mild and a severe influenza viral infection. A subject having a mild influenza viral infection can be associated with a level of IL-17 that is greater than about 50 pg/mL or greater than about 500 pg/mL. The subject having the mild influenza viral infection can be associated with a level of IL-17 that is greater than about 500 pg/mL. A subject having a severe influenza viral infection can be associated with a level of IL-17 that is greater than about 50 pg/mL, but is less than about 500 pg/mL. In other words, the subject having the severe influenza viral infection can be associated with a level of IL-17 between about 50 pg/mL and 500 pg/mL.

The sample obtained from a subject having a mild influenza viral infection can have a higher level of IL-17 as compared to the sample obtained from a subject having a severe influenza viral infection. The sample obtained from the subject having the mild influenza viral infection can have at least about 0.2-fold, at least about 0.3-fold, at least about 0.4-fold, at least about 0.5-fold, at least about 0.6-fold, at least about 0.7-fold, at least about 0.8-fold , at least about 0.9-fold, at least about 1.0-fold, at least about 1.1-fold, at least about 1.2-fold, at least about 1.3-fold, at least about 1.4-fold, at least about 1.5-fold, at least about 1.6-fold, at least about 1.7-fold, at least about 1.8-fold, at least about 1.9 fold, at least about 2.0-fold, at least about 2.1-fold, at least about 2.2-fold, at least about 2.3-fold, at least about 2.4-fold, at least about 2.5-fold, at least about 2.6-fold, at least about 2.7-fold, at least about 2.8-fold, at least about 2.9-fold, at least about 3.0-fold, at least about 3.1-fold, at least about 3.2-fold, at least about 3.3-fold, at least about 3.4-fold, at least about 3.5-fold, at least about 3.6-fold, at least about 3.7-fold, at least about 3.8-fold, at least about 3.9-fold or at least about 4.0-fold higher level of IL-17 as compared to the sample obtained from the subject having the severe influenza viral infection. The sample obtained from the subject having the mild influenza viral infection can have at least about 2.0-fold higher level of IL-17 as compared to the sample obtained from the subject having the severe influenza viral infection.

### 4. Method of Treatment and/or Prevention

The present invention is also directed to a method of increasing an immune response to influenza in a subject by administering the immunotherapeutic composition to the subject. The increased immune response can be used to treat, prevent, and/or protect against disease in the subject, for example, influenza viral infection. The increased immune response can provide about 60% to about 100%, about 65% to about 100%, about 70% to about 100%, about 75% to about 100%, about 80% to about 100%, about 85% to about 100%, about 90% to about 100%, about 95% to about 100%, about 60% to about 95%, about 60% to about 90%, about 60% to about 85%, about 60% to about 80%, about 60% to about 75%, or about 60% to about 70% survival of influenza viral infection. The increased immune response can provide at least about 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% survival of influenza viral infection.

The increased immune response can prevent about 60% to about 100%, about 65% to about 100%, about 70% to about 100%, about 75% to about 100%, about 80% to about 100%, about 85% to about 100%, about 90% to about 100%, about 95% to about 100%, about 60% to about 95%, about 60% to about 90%, about 60% to about 85%, about 60% to about 80%, about 60% to about 75%, or about 60% to about 70% of death from influenza viral infection. The increased immune response can prevent at least about 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of death from influenza viral infection.

The immunotherapeutic composition dose can be between 1 µg to 10 mg active component/kg body weight/time, and can be 20 µg to 10 mg component/kg body weight/time. The immunotherapeutic composition can be administered every 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days. The number of immunotherapeutic composition doses for effective treatment can be 1, 2, 3, 4, 5, 6, 7, 8, 9, or 10.

### a. Influenza Virus

The subject administered the immunotherapeutic composition can have an increased immune response as compared to the subject not administered the immunotherapeutic composition as described above. The increased immune response can be used to treat, prevent, and/or protect against influenza virus. The influenza virus can be an influenza type A, B, or C virus. The influenza virus can be an influenza A virus.

### (1) Influenza A Virus

The influenza virus can be the influenza A virus. The influenza A viruses are divided into subtypes based upon the identity of two surface proteins, namely hemagglutinin (H) and neuraminidase (N). Seventeen and ten different subtypes of hemagglutinin and neuraminidase, respectively, exist. Accordingly, influenza A viruses are identified by respective subtypes of hemagglutinin and neuraminidase. The influenza A virus can be any number of subtypes, for example, but not limited to, H3N2, H1N1, H5N1, and H7N9.

### b. Administration

The immunotherapeutic composition can be formulated in accordance with standard techniques well known to those skilled in the pharmaceutical art. Such compositions can be administered in dosages and by techniques well known to those skilled in the medical arts taking into consideration such factors as the age, sex, weight, and condition of the particular subject, and the route of administration. The subject can be a mammal, such as a human, a horse, a cow, a pig, a sheep, a cat, a dog, a rat, or a mouse.

The immunotherapeutic composition can be administered prophylactically or therapeutically. In prophylactic administration, the immunotherapeutic compositions can be administered in an amount sufficient to induce an immune response. In therapeutic applications, the immunotherapeutic compositions are administered to a subject in need thereof in an amount sufficient to elicit a therapeutic effect. An amount adequate to accomplish this is defined as "therapeutically effective dose." Amounts effective for this use will depend on, e.g., the particular composition of the immunotherapeutic composition regimen administered, the manner of administration, the stage and severity of the disease, the general state of health of the patient, and the judgment of the prescribing physician.

The immunotherapeutic composition can be administered by methods well known in the art as described in Donnelly et al. (Ann. Rev. Immunol. 15:617-648 (1997)); Felgner et al. (U.S. Pat. No. 5,580,859, issued Dec. 3, 1996); Felgner (U.S. Pat. No. 5,703,055, issued Dec. 30, 1997); and Carson et al. (U.S. Pat. No. 5,679,647, issued Oct. 21, 1997), the contents of all of which are incorporated herein by reference in their entirety. The DNA of the immunotherapeutic composition can be complexed to particles or beads that can be administered to an individual, for example, using a immunotherapeutic composition gun. One skilled in the art would know that the choice of a pharmaceutically acceptable carrier, including a physiologically acceptable compound, depends, for example, on the route of administration of the expression vector.

The immunotherapeutic composition can be delivered via a variety of routes. Typical delivery routes include parenteral administration, e.g., intradermal, intramuscular or subcutaneous delivery. Other routes include oral administration, intranasal, and intravaginal routes. For the DNA of the immunotherapeutic composition in particular, the immunotherapeutic composition can be delivered to the interstitial spaces of tissues of an individual (Felgner et al., U.S. Pat. Nos. 5,580,859 and 5,703,055, the contents of all of which are incorporated herein by reference in their entirety). The immunotherapeutic composition can also be administered to muscle, or can be administered via intradermal or subcutaneous injections, or transdermally, such as by iontophoresis. Epidermal administration of the immunotherapeutic composition can also be employed. Epidermal administration can involve mechanically or chemically irritating the outermost layer of epidermis to stimulate an immune response to the irritant (Carson et al., U.S. Pat. No. 5,679,647, the contents of which are incorporated herein by reference in its entirety).

The immunotherapeutic composition can also be formulated for administration via the nasal passages. Formulations suitable for nasal administration, wherein the carrier is a solid, can include a coarse powder having a particle size, for example, in the range of about 10 to about 500 microns which is administered in the manner in which snuff is taken, i.e., by rapid inhalation through the nasal passage from a container of the powder held close up to the nose. The formulation can be a nasal spray, nasal drops, or by aerosol administration by nebulizer. The formulation can include aqueous or oily solutions of the immunotherapeutic composition.

The immunotherapeutic composition can be a liquid preparation such as a suspension, syrup or elixir. The immunotherapeutic composition can also be a preparation for parenteral, subcutaneous, intradermal, intramuscular or intravenous administration (e.g., injectable administration), such as a sterile suspension or emulsion.

The immunotherapeutic composition can be incorporated into liposomes, microspheres or other polymer matrices (Felgner et al., U.S. Pat. No. 5,703,055; Gregoriadis, Liposome Technology, Vols. Ito III (2nd ed. 1993), the contents of which are incorporated herein by reference in their entirety). Liposomes can consist of phospholipids or other lipids, and can be nontoxic, physiologically acceptable and metabolizable carriers that are relatively simple to make and administer.

The immunotherapeutic composition can be administered via electroporation, such as by a method described in U.S. Patent No. 7,664,545, the contents of which are incorporated herein by reference. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359, the contents of which are incorporated herein by reference in their entirety. The electroporation may be carried out via a minimally invasive device.

The minimally invasive electroporation device ("MID") may be an apparatus for injecting the immunotherapeutic composition described above and associated fluid into body tissue. The device may comprise a hollow needle, DNA cassette, and fluid delivery means, wherein the device is adapted to actuate the fluid delivery means in use so as to concurrently (for example, automatically) inject DNA into body tissue during insertion of the needle into the said body tissue. This has the advantage that the ability to inject the DNA and associated fluid gradually while the needle is being inserted leads to a more even distribution of the fluid through the body tissue. The pain experienced during injection may be reduced due to the distribution of the DNA being injected over a larger area.

The MID may inject the immunotherapeutic composition into tissue without the use of a needle. The MID may inject the immunotherapeutic composition as a small stream or jet with such force that the immunotherapeutic composition pierces the surface of the tissue and enters the underlying tissue and/or muscle. The force behind the small stream or jet may be provided by expansion of a compressed gas, such as carbon dioxide through a micro-orifice within a fraction of a second. Examples of minimally invasive electroporation devices, and methods of using them, are described in published U.S. Patent Application No. 20080234655; U.S. Patent No. 6,520,950; U.S. Patent No. 7,171,264; U.S. Patent No. 6,208,893; U.S. Patent NO. 6,009,347; U.S. Patent No. 6,120,493; U.S. Patent No. 7,245,963; U.S. Patent No. 7,328,064; and U.S. Patent No. 6,763,264, the contents of each of which are herein incorporated by reference.

The MID may comprise an injector that creates a high-speed jet of liquid that painlessly pierces the tissue. Such needle-free injectors are commercially available. Examples of needle-free injectors that can be utilized herein include those described in U.S. Patent Nos. 3,805,783; 4,447,223; 5,505,697; and 4,342,310, the contents of each of which are herein incorporated by reference.

A desired immunotherapeutic composition in a form suitable for direct or indirect electrotransport may be introduced (e.g., injected) using a needle-free injector into the tissue to be treated, usually by contacting the tissue surface with the injector so as to actuate delivery of a jet of the agent, with sufficient force to cause penetration of the immunotherapeutic composition into the tissue. For example, if the tissue to be treated is mucosa, skin or muscle, the agent is projected towards the mucosal or skin surface with sufficient force to cause the agent to penetrate through the stratum corneum and into dermal layers, or into underlying tissue and muscle, respectively.

Needle-free injectors are well suited to deliver immunotherapeutic compositions to all types of tissues, particularly to skin and mucosa. In some embodiments, a needle-free injector may be used to propel a liquid that contains the immunotherapeutic composition to the surface and into the subject's skin or mucosa. Representative examples of the various types of tissues that can be treated using the invention methods include pancreas, larynx, nasopharynx, hypopharynx, oropharynx, lip, throat, lung, heart, kidney, muscle, breast, colon, prostate, thymus, testis, skin, mucosal tissue, ovary, blood vessels, or any combination thereof.

The MID may have needle electrodes that electroporate the tissue. By pulsing between multiple pairs of electrodes in a multiple electrode array, for example set up in rectangular or square patterns, provides improved results over that of pulsing between a pair of electrodes. Disclosed, for example, in U.S. Patent No. 5,702,359 entitled "Needle Electrodes for Mediated Delivery of Drugs and Genes" is an array of needles wherein a plurality of pairs of needles may be pulsed during the therapeutic treatment. In that application, which is incorporated herein by reference as though fully set forth, needles were disposed in a circular array, but have connectors and switching apparatus enabling a pulsing between opposing pairs of needle electrodes. A pair of needle electrodes for delivering recombinant expression vectors to cells may be used. Such a device and system is described in U.S. Patent No. 6,763,264, the contents of which are herein incorporated by reference. Alternatively, a single needle device may be used that allows injection of the DNA and electroporation with a single needle resembling a normal injection needle and applies pulses of lower voltage than those delivered by presently used devices, thus reducing the electrical sensation experienced by the patient.

The MID may comprise one or more electrode arrays. The arrays may comprise two or more needles of the same diameter or different diameters. The needles may be evenly or unevenly spaced apart. The needles may be between 0.005 inches and 0.03 inches, between 0.01 inches and 0.025 inches; or between 0.015 inches and 0.020 inches. The needle may be 0.0175 inches in diameter. The needles may be 0.5 mm, 1.0 mm, 1.5 mm, 2.0 mm, 2.5 mm, 3.0 mm, 3.5 mm, 4.0 mm, or more spaced apart.

The MID may consist of a pulse generator and a two or more-needle immunotherapeutic composition injectors that deliver the immunotherapeutic composition and electroporation pulses in a single step. The pulse generator may allow for flexible programming of pulse and injection parameters via a flash card operated personal computer, as well as comprehensive recording and storage of electroporation and patient data. The pulse generator may deliver a variety of volt pulses during short periods of time. For example, the pulse generator may deliver three 15 volt pulses of 100 ms in duration. An example of such a MID is the Elgen 1000 system by Inovio Biomedical Corporation, which is described in U.S. Patent No. 7,328,064, the contents of which are herein incorporated by reference.

The MID may be a CELLECTRA (Inovio Pharmaceuticals, Blue Bell PA) device and system, which is a modular electrode system, that facilitates the introduction of a macromolecule, such as a DNA, into cells of a selected tissue in a body or plant. The modular electrode system may comprise a plurality of needle electrodes; a hypodermic needle; an electrical connector that provides a conductive link from a programmable constant-current pulse controller to the plurality of needle electrodes; and a power source. An operator can grasp the plurality of needle electrodes that are mounted on a support structure and firmly insert them into the selected tissue in a body or plant. The macromolecules are then delivered via the hypodermic needle into the selected tissue. The programmable constant-current pulse controller is activated and constant-current electrical pulse is applied to the plurality of needle electrodes. The applied constant-current electrical pulse facilitates the introduction of the macromolecule into the cell between the plurality of electrodes. Cell death due to overheating of cells is minimized by limiting the power dissipation in the tissue by virtue of constant-current pulses. The Cellectra device and system is described in U.S. Patent No. 7,245,963, the contents of which are herein incorporated by reference.

The MID may be an Elgen 1000 system (Inovio Pharmaceuticals). The Elgen 1000 system may comprise device that provides a hollow needle; and fluid delivery means, wherein the apparatus is adapted to actuate the fluid delivery means in use so as to concurrently (for example automatically) inject fluid, the described immunotherapeutic composition herein, into body tissue during insertion of the needle into the said body tissue. The advantage is the ability to inject the fluid gradually while the needle is being inserted leads to a more even distribution of the fluid through the body tissue. It is also believed that the pain experienced during injection is reduced due to the distribution of the volume of fluid being injected over a larger area.

In addition, the automatic injection of fluid facilitates automatic monitoring and registration of an actual dose of fluid injected. This data can be stored by a control unit for documentation purposes if desired.

It will be appreciated that the rate of injection could be either linear or non-linear and that the injection may be carried out after the needles have been inserted through the skin of the subject to be treated and while they are inserted further into the body tissue.

Suitable tissues into which fluid may be injected by the apparatus of the present invention include tumor tissue, skin or liver tissue but may be muscle tissue.

The apparatus further comprises needle insertion means for guiding insertion of the needle into the body tissue. The rate of fluid injection is controlled by the rate of needle insertion. This has the advantage that both the needle insertion and injection of fluid can be controlled such that the rate of insertion can be matched to the rate of injection as desired. It also makes the apparatus easier for a user to operate. If desired means for automatically inserting the needle into body tissue could be provided.

A user could choose when to commence injection of fluid. Ideally however, injection is commenced when the tip of the needle has reached muscle tissue and the apparatus may include means for sensing when the needle has been inserted to a sufficient depth for injection of the fluid to commence. This means that injection of fluid can be prompted to commence automatically when the needle has reached a desired depth (which will normally be the depth at which muscle tissue begins). The depth at which muscle tissue begins could for example be taken to be a preset needle insertion depth such as a value of 4 mm which would be deemed sufficient for the needle to get through the skin layer.

The sensing means may comprise an ultrasound probe. The sensing means may comprise a means for sensing a change in impedance or resistance. In this case, the means may not as such record the depth of the needle in the body tissue but will rather be adapted to sense a change in impedance or resistance as the needle moves from a different type of body tissue into muscle. Either of these alternatives provides a relatively accurate and simple to operate means of sensing that injection may commence. The depth of insertion of the needle can further be recorded if desired and could be used to control injection of fluid such that the volume of fluid to be injected is determined as the depth of needle insertion is being recorded.

The apparatus may further comprise: a base for supporting the needle; and a housing for receiving the base therein, wherein the base is moveable relative to the housing such that the needle is retracted within the housing when the base is in a first rearward position relative to the housing and the needle extends out of the housing when the base is in a second forward position within the housing. This is advantageous for a user as the housing can be lined up on the skin of a patient, and the needles can then be inserted into the patient's skin by moving the housing relative to the base.

As stated above, it is desirable to achieve a controlled rate of fluid injection such that the fluid is evenly distributed over the length of the needle as it is inserted into the skin. The fluid delivery means may comprise piston driving means adapted to inject fluid at a controlled rate. The piston driving means could for example be activated by a servo motor. However, the piston driving means may be actuated by the base being moved in the axial direction relative to the housing. It will be appreciated that alternative means for fluid delivery could be provided. Thus, for example, a closed container which can be squeezed for fluid delivery at a controlled or non-controlled rate could be provided in the place of a syringe and piston system.

The apparatus described above could be used for any type of injection. It is however envisaged to be particularly useful in the field of electroporation and so it may further comprises means for applying a voltage to the needle. This allows the needle to be used not only for injection but also as an electrode during, electroporation. This is particularly advantageous as it means that the electric field is applied to the same area as the injected fluid. There has traditionally been a problem with electroporation in that it is very difficult to accurately align an electrode with previously injected fluid and so user's have tended to inject a larger volume of fluid than is required over a larger area and to apply an electric field over a higher area to attempt to guarantee an overlap between the injected substance and the electric field. Using the present invention, both the volume of fluid injected and the size of electric field applied may be reduced while achieving a good fit between the electric field and the fluid.

### 5. Treatment Kit

Provided herein is a treatment kit, which can be used for treating a subject using the method of treatment and/or prevention as described above. The treatment kit can comprise the immunotherapeutic composition. The treatment kit can be used with the method of treatment and/or prevention described above. The treatment kit can also comprise one or more containers, such as vials or bottles, with each container containing a separate reagent.

The treatment kit according to the present disclosure preferably includes instructions for carrying out the treatment and/or prevention method of the invention and/or how to use the treatment kit. Instructions included with the treatment kit of the present disclosure can be affixed to packaging material or can be included as a package insert. While instructions are typically written or printed materials, they are not limited to such. Any medium capable of storing instructions and communicating them to an end user is contemplated by this disclosure. Such media include, but are not limited to, electronic storage media (e.g., magnetic discs, tapes, cartridges, and chips), optical media (e.g., CD ROM), and the like. As used herein, the term "instructions" can include the address of an internet set which provides instructions.

### 6. Diagnostic Kit

Provided herein is a diagnostic kit, which can be used for diagnosing influenza viral infection of a subject in need thereof performing the diagnostic method as described above. The diagnostic kit can be used with the method of diagnosis described above. The diagnostic kit can also comprise one or more containers, such as vials or bottles, with each container containing a separate reagent. The diagnostic kit can further include other material(s), which may be desirable from a user standpoint, such as a buffer(s), a diluent(s), a standard(s), and/or any other material useful in sample processing, washing, or conducting any other step of the diagnostic method described herein.

The diagnostic kit according to the present disclosure preferably includes instructions for carrying out the diagnostic method of the invention and/or how to use the diagnostic kit. Instructions included in the diagnostic kit of the present disclosure can be affixed to packaging material or can be included as a package insert. While instructions are typically written or printed materials, they are not limited to such. Any medium capable of storing instructions and communicating them to an end user is contemplated by this disclosure. Such media include, but are not limited to, electronic storage media (e.g., magnetic discs, tapes, cartridges, and chips), optical media (e.g., CD ROM), and the like. As described above, the term "instructions" can include the address of an internet set which provides instructions.

The present invention has multiple aspects, illustrated by the following non-limiting examples.

### 7. Examples

### Example 1

### IL-17A Mediates Resistance to H5N1 Influenza A Viral Infection

To determine whether IL-17A can mediate resistance to influenza A viral infection in vivo, H5N1 influenza A viral infection was examined in C57BL/6 mice and IL-17A knockout mice as described below.

Wild-type C57BL/6 mice were challenged with 5 LD₅₀ of influenza A virus H5N1 (A/Chicken/Henan/1/2004) by nasal administration of the virus. As a control, a second group of wild-type C57BL/6 mice were administered by a nasal route phosphate buffered saline (PBS) instead of the influenza A virus H5N1. 6 days post-infection (dpi), IL-17A protein levels were measured in the serum of challenged and control mice by using BD^{™} Cytometric Bead Array (CBA).

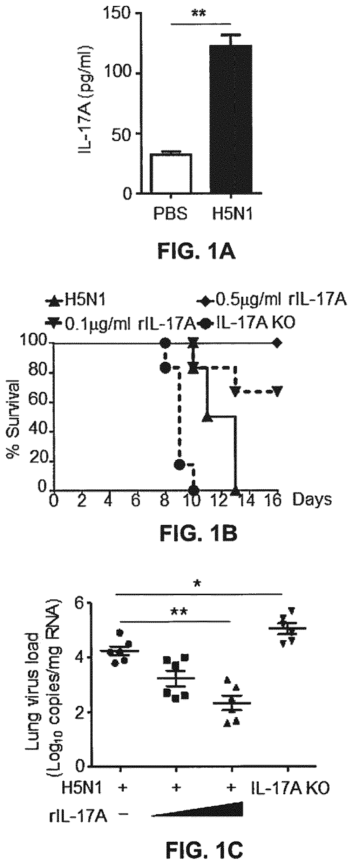

As shown in FIG. 1A, IL-17A protein levels were significantly increased in the challenged mice as compared to the control (unchallenged) mice. The data in FIG. 1A are representative of three independent experiments, the error bars reflect mean ± standard error of the mean (SEM), and **p < 0.01 (unpaired student's t-test). Mice challenged with influenza A virus H5N1 had about 5-fold higher levels of IL-17A as compared to the control mice. Accordingly, this data indicated that IL-17A levels in serum increased in response to H5N1 influenza A viral infection.

To determine if IL-17A mediated protection against influenza A viral infection, two groups (n=6 per group) of wild-type C57BL/6 mice were pre-treated intraperitoneally (i.p.) with 0.1 µg and 0.5 µg, respectively, of recombinant IL-17A (rIL-17A) on day 7 and day 2 before viral infection. rIL-17A is a mouse IL-17A homodimer. A third group (n=6) of wild-type C57BL/6 mice served as a control and did not receive pre-treatment with rIL-17A. A fourth group (n=6) of mice, which were IL-17A knockout mice, also did not receive pre-treatment with rIL-17A. The IL-17A knockout is in the C57BL/6 background. The four groups of mice were infected intranasally with 5 LD₅₀ of influenza A virus H5N1 on day 0 and the mortality of the mice was monitored daily for 16 days following challenge with the virus.

As shown in the FIG. 1B, IL-17A knockout mice were more susceptible to challenge with influenza A virus H5N1 than the non-treated C57BL/6 mice. The data in FIG. 1B are representative of three independent experiments. In particular, the IL-17A knockout mice died at 10 dpi while the untreated C57BL/6 mice died at 13 dpi. In contrast, C57BL/6 mice pre-treated with rIL-17A were protected from challenge with influenza A virus H5N1 in a dose-dependent manner (FIG. 1B). C57BL/6 mice pre-treated with 0.1 µg of rIL-17A were moderately protected and at day 16, about 70% of the mice had survived the challenge with influenza A virus H5N1. C57BL/6 mice pre-treated with 0.5 µg of rIL-17A were normal and at day 16 (i.e., end of the study), 100% of the mice had survived the challenge with influenza A virus H5N1.

To determine if viral load corresponded with the protection afforded by IL-17A, two groups (n=6 per group) of wild-type C57BL/6 mice were pre-treated intraperitoneally (i.p.) with 0.1 µg and 0.5 µg, respectively, of recombinant IL-17A (rIL-17A) on day 7 and day 2 before viral infection. A third group (n=6) of wild-type C57BL/6 mice served as a control and did not receive pre-treatment with rIL-17A. A fourth group (n=6) of mice, which were IL-17A knockout mice, also did not receive pre-treatment with rIL-17A. The four groups of mice were infected intranasally with 5 LD₅₀ of influenza A virus H5N1 on day 0. At 6 dpi, the mice were sacrificed and lung tissue was collected from each mouse. The lung tissue was homogenized and the viral load in the respective lung tissues was determined by quantitative real-time polymerase chain reaction (PCR) analysis. The real-time PCR analysis detected the *HA* gene of the influenza A virus H5N1.

As shown in FIG. 1C, the protection provided by pre-treatment with rIL-17A correlated with the viral load. The data in FIG. 1C are representative of three independent experiments, error bars reflected mean ± SEM, *p < 0.05 (unpaired student's t-test), and **p < 0.01 (unpaired student's t-test). Both groups of C57BL/6 mice administered rIL-17A had lower levels of viral load as compared to C57BL/6 mice that were not pre-treated with rIL-17A. The viral load also corresponded with the dose of rIL-17A administered to the C57BL/6 mice, in which the 0.5 µg dose of rIL-17A resulted in a lower viral load than the 0.1 µg dose of rIL-17A. Additionally, the IL-17A knockout mice, which were more susceptible to challenge with the influenza A virus H5N1 as discussed above, had a higher viral load than the wild-type C57BL/6 mice that were or were not pre-treated with rIL-17A.

In summary, the above data indicated that IL-17A provided protection against (and mediated resistance to) infection with influenza A virus H5N1 because IL-17A knockout mice were more susceptible to viral infection while wild-type mice pre-treated with rIL-17A had reduced viral loads and increased survival in a dose dependent manner. Additionally, these data indicated that IL-17A levels increased upon infection with influenza A virus H5N1, and thus, IL-17A levels reflected influenza viral infection.

### Example 2

### IL-17A Mediates Resistance to H7N9 Influenza A Viral Infection

As described above, IL-17A levels increased in response to infection with influenza A virus H5N1. IL-17A also provided protection against influenza A virus H5N1 infection. To determine if IL-17A mediated resistance to other influenza A viruses in vivo, H7N9 influenza A viral infection was examined in C57BL/6 mice as described below.

Wild-type C57BL/6 mice were challenged with 10 LD₅₀ of influenza A virus H7N9 (A/Shanghai/4664T/2013) by nasal administration of the virus. As a control, a second group of wild-type C57BL/6 mice were administered by a nasal route phosphate buffered saline (PBS) instead of the influenza A virus H7N9. 7 days post-infection (dpi), IL-17A protein levels were measured in the serum of challenged and control mice by by using BD^{™} Cytometric Bead Array (CBA).

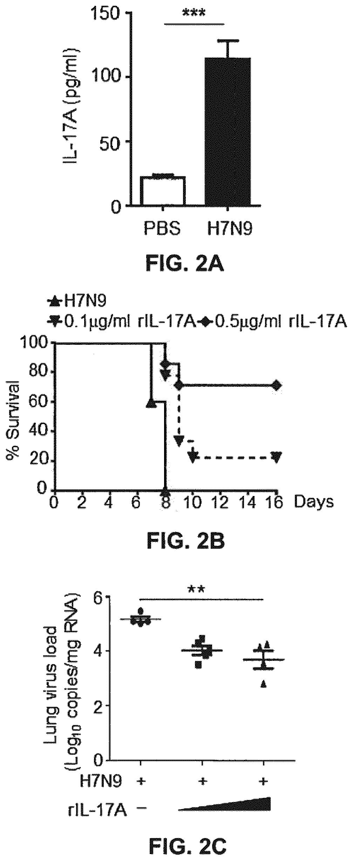

As shown in FIG. 2A, IL-17A protein levels were significantly increased in the challenged mice as compared to the control (unchallenged) mice. The data in FIG. 2A are representative of three independent experiments, the error bars reflected mean ± standard error of the mean (SEM), and ***p < 0.001 (unpaired students t-test). Mice challenged with influenza A virus H7N9 had about 5-fold higher levels of IL-17A as compared to the control mice. This data indicated that IL-17A levels in serum increased in response to H7N9 influenza A viral infection. Accordingly, IL-17A levels in serum increased in response to either H7N9 influenza A viral infection (Example 2) or H5N1 influenza A viral infection (Example 1).

To determine if IL-17A mediated protection against H7N9 influenza A viral infection, two groups of wild-type C57BL/6 mice were pre-treated intraperitoneally (i.p.) with 0.1 µg (n=9) and 0.5 µg (n=7), respectively, of recombinant IL-17A (rIL-17A) on day 7 and day 2 before viral infection. A third group (n=7) of wild-type C57BL/6 mice served as a control and did not receive pre-treatment with rIL-17A. The three groups of mice were infected intranasally with 10 LD₅₀ of influenza A virus H7N9 on day 0 and the mortality of the mice was monitored daily for 16 days following challenge with the virus. Additionally, the body weight of the mice was monitored daily for 16 days following challenge with the virus.

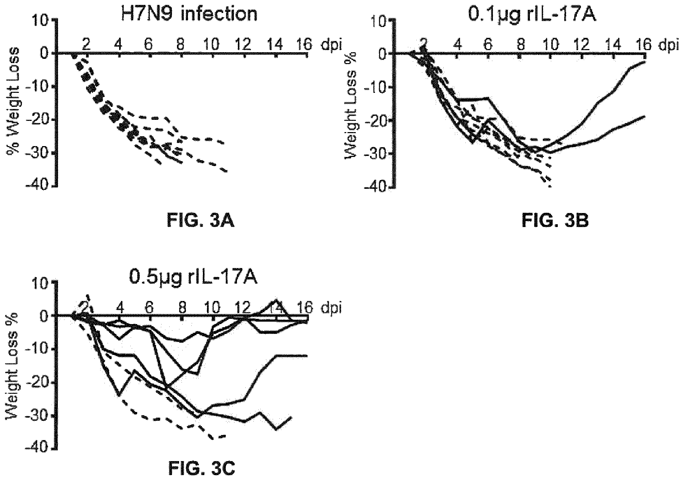

As shown in the FIG. 2B, all C57BL/6 mice that were not pre-treated with rIL-17A and challenged with influenza A virus H7N9 died as early as 8 dpi. The data in FIG. 2B are representative of three independent experiments. In contrast, about 22.2% of the C57BL/6 mice pre-treated with 0.1 µg rIL-17A survived the challenge with influenza A virus H7N9 and were alive at 16 dpi. About 71.4% of the C57BL/6 mice pre-treated with 0.5 µg rIL-17A survived the challenge with influenza A virus H7N9 and were alive at 16 dpi. Additionally, the C57BL/6 mice pre-treated with rIL-17A showed weight loss initially after viral infection, but the surviving mice in both groups (i.e., 0.1 µg and 0.5 µg rIL-17A pre-treatmeant) regained all or most of the lost weight by 16 dpi (FIGS. 3A, 3B, and 3C). As observed with influenza A virus H5N1 above, pre-treatment with rIL-17A provided protection against influenza A virus H7N9 in a dose dependent manner, in which a higher dose of rIL-17A afforded greater protection against (and resistance to) influenza A virus H7N9 infection.

To determine if H7N9 viral load corresponded with the protection afforded by IL-17A, two groups (n=5 per group) of wild-type C57BL/6 mice were pre-treated intraperitoneally (i.p.) with 0.1 µg and 0.5 µg, respectively, of recombinant IL-17A (rIL-17A) on day 7 and day 2 before viral infection. A third group (n=5) of wild-type C57BL/6 mice served as a control and did not receive pre-treatment with rIL-17A. The three groups of mice were infected intranasally with 10 LD₅₀ of influenza A virus H7N9 on day 0. At 7 dpi, the mice were sacrificed and lung tissue was collected from each mouse. The lung tissue was homogenized and the viral load in the respective lung tissues was determined by quantitative real-time polymerase chain reaction (PCR) analysis. The real-time PCR analysis detected the *HA* gene of the influenza A virus H5N1.

As shown in FIG. 2C, the protection provided by pre-treatment with rIL-17A correlated with the viral load. The data in FIG. 2C are representative of three independent experiments, error bars reflect mean ± SEM, and **p < 0.01 (unpaired student's t-test). Both groups of C57BL/6 mice administered rIL-17A had significantly lower levels of viral load as compared to C57BL/6 mice that were not pre-treated with rIL-17A. As observed with influenza A virus H5N1, lower levels of influenza A virus H7N9 were detected in the lung tissue of mice that were pre-treated with rIL-17A in a dose dependent manner. Accordingly, pre-treatment with IL-17A provided protection against (and mediated resistance to) both influenza A viruses H5N1 and H7N9, and lowered viral loads of both viruses.

In summary, the above data indicated that IL-17A provided protection against (and mediated resistance to) infection with influenza A virus H7N9 because mice pre-treated with rIL-17A had reduced viral loads and increased survival in a dose dependent manner. Additionally, these data indicated that IL-17A levels increased upon infection with influenza A virus H7N9, and thus, IL-17A levels reflected viral infection. Accordingly, the data of Examples 1 and 2 demonstrated that IL-17A provided protection against (and mediated resistance to) infection with multiple influenza A viruses, namely H5N1 and H7N9, and IL-17A levels reflected infection with either influenza A virus.

### Example 3

### Pulmonary Inflammatory Response in Mice Pre-Treated with rIL-17A

Upon infection with influenza A virus H5N1 and H7N9, lymphocytes infiltrate lung tissue in large numbers, which contributes to inflammation, tissue injury, and death. Histochemistry and immunohistochemistry were employed to examine CD8⁺ T cell infiltration in the lung tissue of mice that were or were not pre-treated with rIL-17A and challenged with influenza A virus H5N1 or H7N9.

Specifically, one group of C57BL/6 mice was pre-treated i.p. with 0.5 µg of rIL-17A on day 7 and day 2 before viral infection. A second group of C57BL/6 mice did not receive pre-treatment with rIL-17A. Both the first and second groups of C57BL/6 mice were infected intranasally with 5 LD₅₀ of influenza A virus H5N1 on day 0. A third group of C57BL/6 mice served as a naive control and thus, did not receive pre-treatment with rIL-17A and was not administered influenza A virus H5N1. At 6 dpi, the mice from all three groups were sacrificed and lung tissue sections were obtained from the mice. Tissue sections were stained with hematoxylin and eosin (H&E). Immunohistochemistry was also performed on tissue sections with an antibody specific for CD8⁺ T cells.

The top panels in FIG. 4A show H&E (top, left panel) and CD8⁺ T cell (top, right panel) staining of representative lung tissue sections from the naive mice. The native mice served as a control for the appearance of normal or healthy lung tissue. The lung tissue from mice challenged with influenza A virus H5N1 exhibited a thickening of alveolar walls and a wide distribution of CD8⁺ T cells (FIG. 4A, middle left and right panels show the H&E and CD8⁺ T cell staining, respectively, of representative lung tissue sections from mice challenged with influenza A virus H5N1). The lung tissue from mice challenged with influenza A virus H5N1 also showed severe bronchopneumonia in these mice.

In contrast, the lung tissue from mice pre-treated with rIL-17A before challenge with influenza A virus H5N1 exhibited mild injury and a discontinuous spatial distribution of infiltrating CD8⁺ T cells (FIG. 4A, bottom left and right panels show the H&E and CD8⁺ T cell staining, respectively, of representative lung tissue sections from mice pre-treated with rIL-17A before challenge with influenza A virus H5N1). In other words, less infiltration of CD8⁺ T cells occurred in the lung tissue of pre-treated, challenged mice as compared to challenged mice, and thus, the lung tissue of pre-treated, challenged mice exhibited less tissue injury and inflammation as compared to the lung tissue of challenged mice. Together, these data indicated that pre-treatment with rIL-17A decreased CD8⁺ T cell infiltration of lung tissue in response to challenge with influenza A virus H5N1, thereby decreasing injury to and inflammation of the same lung tissue.

Additionally, CD8⁺ T cell infiltration into lung tissue was examined in mice challenged with influenza A virus H7N9. Specifically, one group of C57BL/6 mice was pre-treated i.p. with 0.5 µg of rIL-17A on day 7 and day 2 before viral infection. A second group of C57BL/6 mice did not receive pre-treatment with rIL-17A. Both the first and second groups of C57BL/6 mice were infected intranasally with 10 LD₅₀ of influenza A virus H7N9 on day 0. A third group of C57BL/6 mice served as a naive control and thus, did not receive pre-treatment with rIL-17A and was not administered influenza A virus H7N9. At 6 dpi, the mice from all three groups were sacrificed and lung tissue sections were obtained from the mice. Tissue sections were stained with H&E. Immunohistochemistry was also performed on tissue sections with an antibody specific for CD8⁺ T cells.

The top panels in FIG. 4B show H&E (top, left panel) and CD8⁺ T cell (top, right panel) staining of representative lung tissue sections from naive mice. As described above, the naive mice served as a control for the appearance of normal or healthy lung tissue. The lung tissue from mice challenged with influenza A virus H7N9 also exhibited a thickening of alveolar walls and a wide distribution of CD8⁺ T cells (FIG. 4B, middle left and right panels show the H&E and CD8⁺ T cell staining, respectively, of representative lung tissue sections from mice challenged with influenza A virus H7N9). The lung tissue from mice challenged with influenza A virus H7N9 also showed severe bronchopneumonia in these mice.

In contrast, the lung tissue from mice pre-treated with rIL-17A before challenge with influenza A virus H7N9 exhibited mild injury and a discontinuous spatial distribution of infiltrating CD8⁺ T cells (FIG. 4B, bottom left and right panels show the H&E and CD8⁺ T cell staining, respectively, of representative lung tissue sections from mice pre-treated with rIL-17A before challenge with influenza A virus H7N9). In other words, less infiltration of CD8⁺ T cells occurred in the lung tissue of pre-treated, challenged mice as compared to challenged mice, and thus, the lung tissue of pre-treated, challenged mice exhibited less tissue injury and inflammation as compared to the lung tissue of challenged mice. Together, these data indicated that pre-treatment with rIL-17A decreased CD8⁺ T cell infiltration of lung tissue in response to challenge with influenza A virus H7N9, thereby decreasing injury to and inflammation of the same lung tissue.

In summary, mice challenged with influenza A virus H5N1 or H7N9 exhibited significant infiltration of lung tissue by CD8⁺ T cells, lung tissue injury, lung tissue inflammation, and severe bronchopneumonia. Pre-treatment with rIL-17A protected the mice challenged with influenza A virus H5N1 or H7N9 because CD8⁺ T cell infiltration of lung tissue was decreased in these mice relative to mice that were not pre-treated with rIL-17A before viral challenge, and thus, the protected mice had reduced lung tissue injury and inflammation. Accordingly, IL-17A provided protection against (and mediated resistance to) to H5N1 and H7N9 viral infection by reducing CD8⁺ T cell infiltration of lung tissue, which would otherwise have contributed to lung tissue injury and inflammation.

### Example 4

### IL-17A Levels in Human Subjects Infected with an Influenza A Virus

Examples 1-3 above demonstrated that IL-17A protected mice against infection with influenza A viruses H5N1 and H7N9. Additionally, IL-17A levels were elevated in those mice infected with influenza A viruses H5N1 and H7N9. To determine if IL-17A levels are altered in human subjects infected with an influenza A virus, IL-17A levels were measured in serum obtained from healthy human subjects and human subjects infected with influenza A virus H3N2, H5N1, H1N1, or H7N9.

Serum samples were collected and analyzed from 50 healthy human subjects and 99 human subjects acutely infected with influenza A virus. In particular, IL-17A levels in the collected sera were measured by using BD^{™} Cytometric Bead Array (CBA). 16 human subjects were infected with influenza A virus H3N2 (i.e., seasonal flu). 65 human subjects were infected with influenza A virus H1N1 (i.e., pandemic flu), of which 16 and 49 human subjects were identified as having severe and mild infections, respectively. 18 human subjects were infected with influenza A virus H7N9, of which 10 and 8 human subjects were identified as having severe and mild infections, respectively.

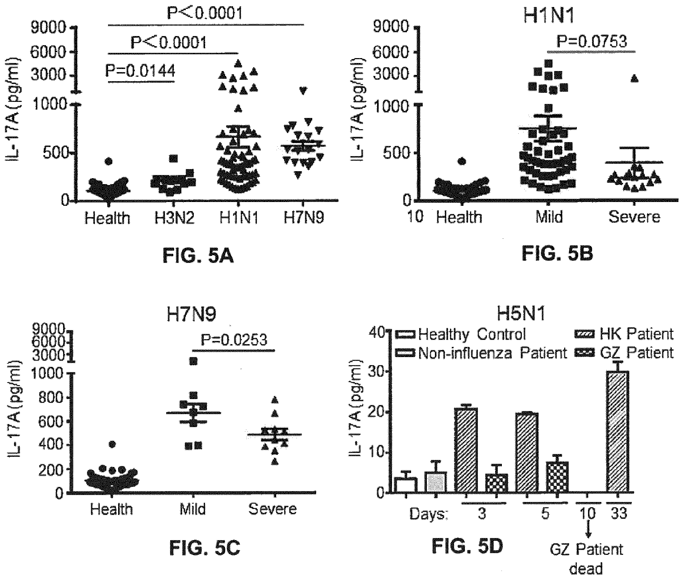

Compared to the healthy subjects, levels of IL-17A were significantly elevated in sera collected from subjects infected with either influenza A virus H1N1 or H7N9 (p<0.0001; FIG. 5A, in which each symbol represented an individual subject, data are mean ± SEM, and the horizontal line represented the respective mean for each group of subjects). Sera from healthy subjects had levels of IL-17A that were less than 50 pg/mL while sera from subjects infected with influenza A virus H1N1, H7N9, or H3N2 had levels of IL-17A that were greater than 50 pg/mL. In particular, H1N1 infected subjects and H7N9 infected subjects had on average 16-fold and 12-fold higher levels, respectively of IL-17A as compared to healthy subjects. Levels of IL-17A were also significantly increased in the sera collected from subjects inflected with influenza A virus H3N2 as compared to healthy subjects (p<0.05, FIG. 5A). H3N2 infected subjects had on average 2-fold higher levels of IL-17A in sera as compared to healthy subjects.

The elevated levels of IL-17A observed in the sera of infected subjects corresponded with the severity of viral infection. Subjects with a mild infection (i.e., clinically mild subjects) displayed only flu-like symptoms and had normal chest X-ray and/or lung computed tomography (CT) scan. Subjects with a severe infection, (i.e., clinically severe subjects) had (1) a fever for more than 3 days, (2) acute cough, sometimes with sputum, blood, and/or chest pain; (3) difficulty in breathing and cyanosis; (4) malaise, slow response, drowsiness, and/or convulsion; (5) vomiting, and/or diarrhea, sometimes resulting in dehydration; (6) chest X-ray and/or lung CT scan showing multi-leaf consolidation and severe pneumonia; (7) respiratory failure or shock and/or multi-organ failure; and/or (8) admittance to an intensive care unit.

For subjects infected with either influenza A virus H1N1 or H7N9, clinically "mild" subjects exhibited a higher level of IL-17A as compared to clinically "severe" subjects (FIGS. 5B and 5C, in which each symbol represented an individual subject, data are mean ± SEM, and the horizontal line represented the respective mean for each group of subjects). Mild subjects had about 2-fold higher levels of IL-17A in sera as compared to subjects having severe infection. In particular, sera from mild subjects had levels of IL-17A greater than 500 pg/mL while sera from severe subjects had levels of IL-17A greater than 50 pg/mL, but less than 500 pg/mL.

With regards to H5N1 viral infection, an enzyme-linked immunosorbent assay was used to detect IL-17A levels in sera collected from 2 subjects infected with influenza A virus H5N1 on day 3, day 5, and day 10 after confirmed infection. One infected subject was from Guangzhoua, China (GK subject) and the second infected subject was from Hong Kong, China (HK subject). As a control, serum samples were also collected from 3 healthy subjects and 1 subject having a viral infection other than influenza (i.e., non-influenza subject).

The GK subject was hospitalized and died 10 days later due to severe lung dysfunction. The GZ subject had a low serum level of IL-17A during the course of infection (FIG. 5D, in which data are mean ± SEM). In contrast, the HK subject had an elevated level of IL-17A as compared to healthy subjects, the non-influenza subject, and the GZ subject (FIG. 5D). The HK subject recovered from the H5N1 viral infection.

In summary, these dated indicated that IL-17A levels were increased or elevated in human subjects infected with an influenza A virus (i.e., H3N2, H1N1, H7N9, and H5N1) as compared to healthy subjects. Accordingly, IL-17A levels increased in humans (Example 4) and mice (Examples 1 and 2) upon infection with the influenza A virus, and therefore, elevated IL-17A levels indicated infection with the influenza A virus. IL-17A levels also corresponded with the severity of infection in the human subjects, in which IL-17A levels were higher in subjects having a mild viral infection as compared to subjects having a severe viral infection.

### Example 5

### IL-6 and TNF-α are not required for Protection against Viral Infection

IL-17A may induce cytokines that are part of the innate immune response, for example, interleukin-6 (IL-6) and tumor necrosis factor alpha (TNF-α). IL-6 and TNF-α may mediate inflammation. To examine if IL-17A mediated resistance to influenza A viral infection via IL-6 and/or TNF-α, the levels of IL-6 and TNF-α were measured in sera collected from mice challenged with influenza A virus H5N1.

Specifically, two groups of C57BL/6 mice were pre-treated i.p. with 0.1 µg and 0.5 µg, respectively, of rIL-17A on day 7 and day 2 before viral infection. The two groups of mice were challenged with 5 LD₅₀ of influenza A virus H5N1 on day 0. The H5N1 virus was administered nasally to the mice. A third group of C57BL/6 mice served as a control and was not pre-treated with rIL-17A nor challenged with influenza A virus H5N1 (i.e., naive mice). At 6 dpi, sera were collected from the mice and IL-6 and TNF-α levels were measured in the collected sera.

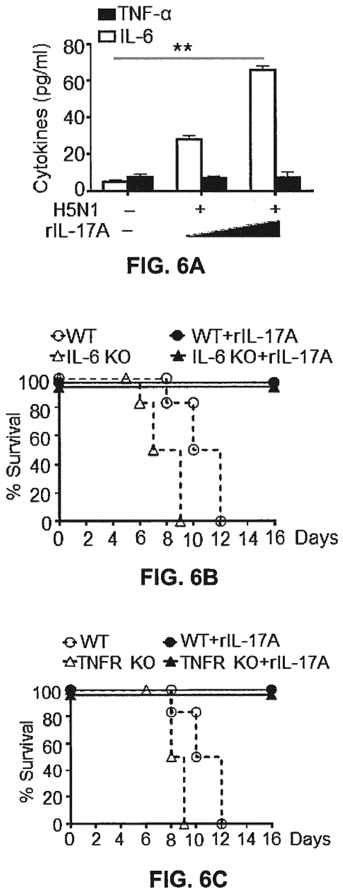

Compared to naive mice, mice pre-treated with rIL-17A before viral challenge had increased levels of IL-6 in the sera (FIG. 6A, **p<0.01). The increase in IL-6 levels was dependent upon the dose of rIL-17A given in the pre-treatment, in which a higher dose of rIL-17A resulted in higher serum levels of IL-6. TNF-α levels, however, were unaltered by pre-treatment with rIL-17A and challenge with influenza A virus H5N1.

Additionally, wild-type (i.e.,C57BL/6), IL-6 knockout, and TNFR1/2 (i.e., TNF-α) knockout mice were challenged with influenza A virus H5N1 alone or in combination with rIL-17A pre-treatment. Mortality after infection was then measured. As shown in FIG. 6B, wild-type mice had died by about 9 dpi while IL-6 knockout mice died by 12 dpi. 100% of the wild-type and IL-6 knockout mice pre-treated with rIL-17A, however, survived the challenge with influenza A virus H5N1. These data indicated that while IL-6 levels increased in a dose dependent manner after pre-treatment with rIL-17A, IL-6 was not required for IL-17A-mediated protection against viral challenge.

As shown in FIG. 6C, wild-type and TNFR1/1 knockout mice had died by 9 dpi and 12 dpi, respectively. 100% of the wild-type and TNFR1/2 knockout mice pre-treated with rIL-17A survived the challenge with influenza A virus H5N1. These data indicated that TNF-α levels do not change in response to pre-treatment with rIL-17A nor is TNF-α required for IL-17A-mediated protection against viral challenge.

In summary, IL-17A provided protection against (and mediated resistance to) H5N1 viral infection independent of IL-6 and TNF-α.

### Example 6

### IFN-γ, but not IL-4, is required for Protection against Viral Infection

Cytokines, for example, interleukin-4 (IL-4) and interferon-gamma (IFN-γ), may control infection by influenza A virus. To examine if IL-17A mediated resistance to influenza A viral infection via IL-4 and/or **IFN-γ,** the levels of IL-4 and IFN-γ were measured in sera collected from mice challenged with influenza A virus H5N1 or H7N9.

Specifically, two groups of C57BL/6 mice were pre-treated i.p. with 0.1 µg and 0.5 µg, respectively, of rIL-17A on day 7 and day 2 before viral infection. A third group of C57BL/6 mice did not receive rIL-17A pre-treatment. The three groups of mice were challenged with 5 LD₅₀ of influenza A virus H5N1 on day 0. The H5N1 virus was administered nasally to the mice. A fourth group of C57BL/6 mice served as a control and was not pre-treated with rIL-17A nor challenged with influenza A virus H5N1 (i.e., naive mice). At 6 dpi, sera were collected from the mice and IL-4 and IFN-γ levels were measured in the collected sera.

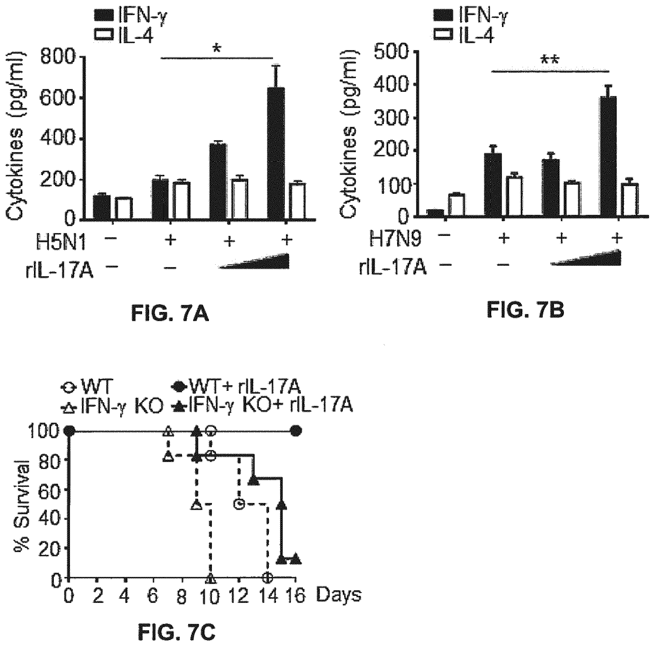

As shown in FIG. 7A, no significant difference in IL-4 levels was observed between the four groups of mice. The data in FIG. 7A are representative of three independent experiments. IFN-γ levels were increased in mice challenged with influenza A virus H5N1 as compared to naive mice. IFN-γ levels, however, significantly increased in a dose dependent manner in response to rIL-17A pre-treatment as compared to mice challenged with influenza A virus H5N1 (FIG. 7A, *p < 0.05 (unpaired student's t-test)). Mice pre-treated with 0.5 µg of rIL-17A had higher serum levels of IFN-γ than mice pre-treated with 0.1 ug of rIL-17A, mice challenged with H5N1, and naive mice (FIG. 7A, about 1.6-fold, about 3.25-fold, and about 6.5-fold, respectively, higher IFN-γ levels). These data indicated that IFN-γ, but not IL-4, levels in serum significantly increased in response to rIL-17A pre-treatment in a dose dependent manner.

To further examine IL-4 and IFN-γ levels, the above experiment was repeated, but with influenza A virus H7N9 instead of influenza A virus H5N1. Specifically, two groups of C57BL/6 mice were pre-treated i.p. with 0.1 µg and 0.5 µg, respectively, of rIL-17A on day 7 and day 2 before viral infection. A third group of C57BL/6 mice did not receive rIL-17A pre-treatment. The three groups of mice were challenged with 10 LD₅₀ of influenza A virus H7N9 on day 0. The H7N9 virus was administered nasally to the mice. A fourth group of C57BL/6 mice served as a control and was not pre-treated with rIL-17A nor challenged with influenza A virus H7N9 (i.e., naive mice). At 6 dpi, sera were collected from the mice and IL-4 and IFN-γ levels were measured in the collected sera.

As shown in FIG. 7B, no difference in IL-4 levels was observed between the four groups of mice. The data in FIG. 7B are representative of three independent experiments. IFN-γ levels were comparable between mice challenged with influenza A virus H7N9 and mice pre-treated with 0.1 µg rIL-17 before viral challenge. IFN-γ levels, however, were significantly increased in mice pre-treated with 0.5 µg rIL-17A as compared to mice not receiving pre-treatment and mice pre-treated with 0.1 µg rIL-17A (FIG. 7B, **p < 0.01 (unpaired student's t-test)). In particular, mice pre-treated with 0.5 µg rIL-17A had about 1.9-fold higher levels of IFN-γ as compared to mice not receiving pre-treatment with rIL-17A. These data indicated that IFN-γ, but not IL-4, levels in serum significantly increased in response to rIL-17A pre-treatment in a dose dependent manner.

To further examine IFN-γ, wild-type (i.e., C56BL/6) and IFN-γ knockout mice were challenged with influenza A virus H5N1 alone or in combination with rIL-17A pre-treatment. Specifically, one group of wild-type mice (n=6) and one group of IFN-γ knockout mice (n=6) were pre-treated i.p. with 0.5 µg of rIL-17A on day 7 and day 2 before viral infection. A second group of wild-type mice (n=6) and a second group of IFN-γ knockout mice (n=6) did not receive rIL-17A pre-treatment. The four groups of mice were challenged with 5 LD₅₀ of influenza A virus H5N1 on day 0. The H5N1 virus was administered nasally to the mice. Mice were monitored daily for mortality 16 days after infection.

As shown in FIG. 7C, wild-type and IFN-γ knockout mice challenged with influenza A virus H5N1 died by 14 dpi and 10 dpi, respectively. This data indicated that the IFN-γ knockout mice were more susceptible to H5N1 viral infection than wild-type mice. Additionally, pre-treatment of the IFN-γ knockout mice with rIL-17A delayed death as compared to the IFN-γ knockout mice that did not receive rIL-17A pre-treatment. By 16 dpi, about 15% of the IFN-γ knockout mice pre-treated with rIL-17A had survived challenge with influenza A virus H5N1. In contrast, 100% of wild-type mice pre-treated with rIL-17A survived the challenge with influenza A virus H5N1. These data indicated that IL-17A mediated resistance to influenza A viral infection through IFN-γ. In other words, IFN-γ was needed for effective protection against influenza A viral infection.

In summary, IL-4 levels were unchanged in response to rIL-17A pre-treatment. IFN-γ levels, however, increased in response to rIL-17A pre-treatment in a dose dependent manner and IL-17A protected against influenza A viral infection via IFN-γ

### Example 7

### IFN-γ produced by CD8⁺ T cells and Natural Killer Cells

During viral infection, IFN-γ may be secreted by CD4⁺ T cells, CD8⁺ T cells, and/or natural killer (NK) cells. To determine the cell population(s) responsible for the increased levels of IFN-γ induced by rIL-17A pre-treatment, IFN-γ positive cells were detected at 6 dpi.

Specifically, wild-type mice (i.e., C57BL/6) mice were pre-treated i.p. with 0.5 µg of rIL-17A on day 7 and day 2 before viral infection. A second group of C57BL/6 mice did not receive rIL-17A pre-treatment. These two groups of wild-type mice plus a group of IL-17A knockout mice were challenged with 5 LD₅₀ of influenza A virus H5N1 on day 0. The H5N1 virus was administered nasally to the mice. Another group of C57BL/6 mice served as a control and was not pre-treated with rIL-17A nor challenged with influenza A virus H5N1 (i.e., naive mice). Splenocytes were isolated from the four groups of mice at 6 dpi and stimulated with formalin-inactivated influenza A virus H5N1 for 12 hours (h) in vitro. Secretion of IFN-γ by CD4⁺ T cells, CD8⁺ T cells, or NK cells was measured by flow cytometry, which detected intracellular staining of IFN-γ.

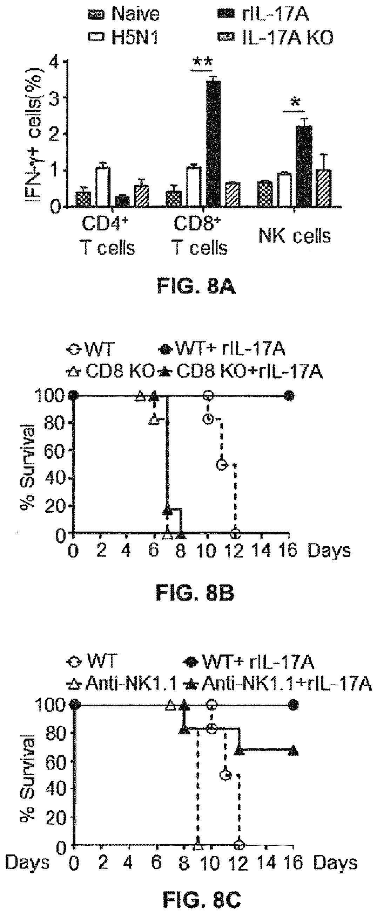

As shown in FIG. 8A, secretion of IFN-γ from CD4⁺ T cells isolated from C57BL/6 mice pre-treated with rIL-17A (and challenged with virus) was decreased as compared to IFN-γ secretion from CD4⁺ T cells isolated from mice not receiving rIL-17A pre-treatment (but challenged with virus). Accordingly, secretion of IFN-γ from CD4⁺ T cells decreased in response to rIL-17A pre-treatment. The data in FIG. 8A are representative of three independent experiments. In contrast, rIL-17A pre-treatment significantly increased IFN-γ secretion from CD8⁺ T cells as compared to in the absence of rIL-17A pre-treatment (FIG. 8A, ** p < 0.01). rIL-17A pre-treatment also significantly increased IFN-γ secretion from NK cells as compared to in the absence of rIL-17A pre-treatment (FIG. 8A, *p < 0.05). Accordingly, these data indicated that CD8⁺ T cells and NK cells significantly contributed to the increased levels of IFN-γ observed in response to rIL-17A pre-treatment.

Because CD8⁺ T cells secreted IFN-γ in response to rIL-17A pre-treatment, the ability of mice lacking CD8⁺ T cells (i.e., CD8 knockout mice) to withstand challenge with influenza A virus H5N1 was examined to determine if IL-17A protected against viral infection via CD8⁺ T cells. Specifically, wild-type (i.e., C57BL/6) mice and CD8 knockout mice were pre-treated i.p. with 0.5 µg of rIL-17A on day 7 and day 2 before viral infection. A second group of C57BL/6 mice and a second group of CD8 knockout mice did not receive rIL-17A pre-treatment. The four groups of mice were challenged with 5 LD₅₀ of influenza A virus H5N1 on day 0. The H5N1 virus was administered nasally to the mice. Mice were monitored daily for 16 days after infection for mortality.

As shown in FIG. 8B, wild-type and CD8 knockout mice challenged with influenza A virus H5N1 died at 12 dpi and 7 dpi, respectively. This data indicated that CD8 knockout mice were more susceptible to H5N1 viral infection than wild-type mice. Additionally, CD8 knockout mice pre-treated with rIL-17A died by 8 dpi, and thus, pre-treatment of CD8 knockout mice with rIL-17A delayed death by 1 day as compared to CD8 knockout mice that did not receive rIL-17A pre-treatment (FIG. 8B). As observed above, 100% of wild-type mice pre-treated with rIL-17A survived the challenge with influenza A virus H5N1. These data indicated that IL-17A mediated resistance to influenza A viral infection through CD8⁺ T cells. In other words, CD8⁺ T cells were needed for effective protection against influenza A viral infection.

As demonstrated above, IFN-γ was also secreted by NK cells in response to rIL-17A pre-treatment. Accordingly, the ability of mice depleted ofNK cells (i.e., anti-NK 1.1 mice) to withstand challenge with influenza A virus H5N1 was examined to determine if IL-17A protected against viral infection via NK cells. Specifically, four groups of C57BL/6 mice were examined. The first group of C57BL/6 mice were not depleted of NK cells nor pre-treated with rIL-17A. The second group of C57BL/6 mice was pre-treated i.p. with 0.5 µg rIL-17A on day 7 and day 2 before viral infection. The third group of C57BL/6 mice was injected i.p. with anti-NK 1.1 neutralizing monoclonal antibody on day 7, day 3, and day 1 before viral infection. The fourth group of C57BL/6 mice was injected i.p. with anti-NK 1.1 neutralizing monoclonal antibody on day 7, day 3, and day 1 before viral infection, and pre-treated i.p. with 0.5 µg of rIL-17A on day 7 and day 2 before viral infection. The four groups of mice were challenged with 5 LD₅₀ of influenza A virus H5N1 on day 0. The H5N1 virus was administered nasally to the mice. Mice were monitored daily for 16 days after infection for mortality.

As shown in FIG. 8C, wild-type and NK depleted (i.e., anti-NK 1.1) mice challenged with influenza A virus H5N1 died by 12 dpi and 9 dpi, respectively. This data indicated that NK depleted mice were more susceptible to H5N1 viral infection than wild-type mice. By 16 dpi, about 70% of NK depleted mice pre-treated with rIL-17A (i.e., anti-NK 1.1 + rIL-17A) had survived the challenge with influenza A virus H5N1 (FIG. 8C). 100% of wild-type mice pre-treated with rIL-17A survived challenge with influenza A virus H5N1. These data indicated that NK cells played a smaller role (than CD8⁺ T cells) in facilitating IL-17A-mediated protection against viral infection because mice depleted of NK cells, but pre-treated with rIL-17A had a modestly reduced survival rate as compared to wild-type mice pre-treated with rIL-17A (i.e., about 70% vs. 100% survival rate to viral challenge).

In summary, IFN-γ secretion by CD8⁺ T cells and NK cells, but not CD4⁺ T cells, was significantly increased in response to rIL-17A pre-treatment. The protection afforded by rIL-17A pre-treatment was significantly reduced in the absence of CD8⁺ T cells, but modestly reduced in the absence of NK cells. Rather, in the absence of CD8⁺ T cells, rIL-17A pre-treatment delayed, but did not prevent, death. Together, these data indicated that IFN-γ producing CD8⁺ T cells significantly contributed to IL-17A-mediated protection (and resistance to) influenza A viral infection.

### Example 8

### Adoptive Transfer of CD8⁺ T cells

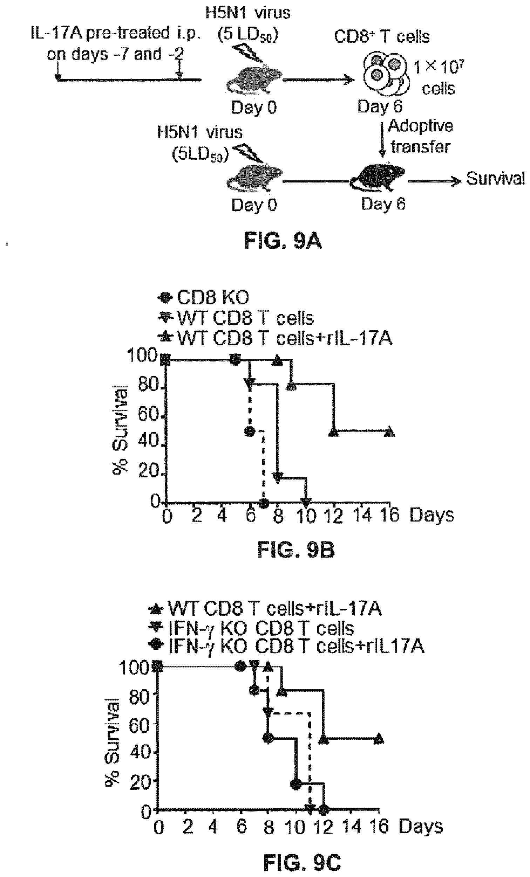

As demonstrated above, IL-17A provided protection against (and mediated resistance to) influenza A viral infection through IFN-γ producing CD8⁺ T cells. As described in more detail below, adoptive transfer was used to further establish that IL-17A-mediated protection is facilitated by IFN-γ producing CD8⁺ T cells. The scheme for adoptive transfer of CD8⁺ T cells is illustrated in FIG. 9A.

In particular, wild-type CD8⁺ T cells from rIL-17A pre-treated, H5N1 infected mice or untreated, H5N1 infected mice were adoptively transferred into H5N1 infected, wild-type mice. Briefly, a first group of C57BL/6 (i.e., wild-type) mice was pre-treated i.p. with 0.5 µg of rIL-17A on day 7 and day 2 before viral infection. A second group of C57BL/6 mice was not pre-treated with rIL-17A. A third group of mice, namely mice lacking CD8⁺ T cells (i.e., CD8 knockout mice), was used as a control and also was not pre-treated with rIL-17A. The three groups of C57BL/6 mice were challenged with 5 LD₅₀ of influenza A virus H5N1 on day 0. C57BL/6 mice that were to receive the adoptive transfer (i.e., recipient mice) were also challenged with 5 LD₅₀ of influenza A virus H5N1 on day 0. The H5N1 virus was administered nasally to the mice.

At 6 dpi, CD8⁺ T cells were isolated from the C57BL/6 mice pre-treated with rIL-17A (i.e., the first group of mice above) and from the C57BL/6 mice that did not receive rIL-17A pre-treatment (i.e., the second group of mice above). 1 x 10⁷ of the CD8⁺ T cells isolated from the first group of mice were then adoptively transferred into C57BL/6 (i.e., wild-type) mice that had been infected with influenza A virus H5N1 for 6 days and mortality was monitored daily until 16 dpi for these recipient mice (i.e., WT CD8 T cells + rIL-17A in FIG. 9B). Additionally, 1 x 10⁷ of the CD8⁺ T cells isolated from the second group of mice were adoptively transferred into C57BL/6 (i.e., wild-type) mice that had been infected with influenza A virus H5N1 for 6 days and mortality was monitored daily until 16 dpi for these recipient mice (i.e., WT CD8+ T cells in FIG. 9B). The CD8 knockout mice were also monitored daily for mortality until 16 dpi (i.e., CD8 KO in FIG. 9B).

As shown in FIG. 9B (and observed in Example 7 and FIG. 8B), the CD8 knockout mice died at 7 dpi and served as a control for mice lacking CD8⁺ T cells. The data in FIG. 9B are representative of three independent experiments. The infected C57BL/6 mice, which received CD8⁺ T cells from the second group of mice (i.e., C57BL/6 mice not receiving rIL-17A pre-treatment, but challenged with influenza A H5N1 virus), died at 10 dpi (FIG. 9B, WT CD8 T cells). In contrast, 50% of the infected C57BL/6 mice, which received CD8⁺ T cells from the first group of mice (i.e., C57BL/6 mice pre-treated with rIL-17A before challenge with influenza A H5N1 virus), survived at 16 dpi (FIG. 9B, WT CD8 T cells + rIL-17A). These data indicated that the CD8⁺ T cells isolated from mice pre-treated with rIL-17A provided the recipient mice protection against (and mediated resistance to) viral infection. These data also indicated that CD8⁺ T cells, upon rIL-17A treatment, are able to provide protection against influenza A viral infection, even in mice already infected with the virus.

Additionally, CD8⁺ T cells from IFN-γ knockout mice receiving or not receiving rIL-17A pre-treatment before viral challenge were adoptively transferred into wild-type, H5N1 infected mice to further establish that IFN-γ producing CD8⁺ T cells facilitated IL-17A-mediated protection against influenza A viral infection. Briefly, C57BL/6 (i.e., wild-type) mice were pre-treated i.p. with 0.5 µg of rIL-17A on day 7 and day 2 before viral infection. A first group of IFN-γ knockout mice was also pre-treated i.p. with 0.5 µg of rIL-17A on day 7 and day 2 before viral infection. A second group of IFN-γ knockout mice was not pre-treated with rIL-17A. The two groups of IFN-γ knockout mice and the rIL-17A pre-treated C57BL/6 mice were challenged with 5 LD₅₀ of influenza A virus H5N1 on day 0. C57BL/6 (i.e., wild-type) mice that were to receive the adoptive transfer (i.e., recipient mice) were also challenged with 5 LD₅₀ of influenza A virus H5N1 on day 0. The H5N1 virus was administered nasally to the mice.

At 6dpi, CD8⁺ T cells were isolated from the C57BL/6 mice pre-treated with rIL-17A, and 1 x 10⁷ of these isolated cells were adoptively transferred into C57BL/6 mice that had been infected with influenza A virus H5N1 for 6 days. The mortality of these recipient mice (i.e., WT CD8 T cells + rIL-17A in FIG. 9C) was monitored daily until 16 dpi.

Also at 6 dpi, CD8⁺ T cells were isolated from the IFN-γ knockout mice pre-treated with rIL-17A (i.e., the first group of IFN-γ knockout mice above) and from the IFN-γ knockout mice that did not receive rIL-17A pre-treatment (i.e., the second group of IFN-γ knockout mice above). 1 x 10⁷ of the CD8⁺ T cells isolated from the first group of IFN-γ knockout mice were then adoptively transferred into C57BL/6 (i.e., wild-type) mice that had been infected with influenza A virus H5N1 for 6 days. The mortality of these recipient mice was monitored daily until 16 dpi (i.e., IFN-γ KO CD8 T cells + rIL-17A in FIG. 9C). Additionally, 1 x 10⁷ of the CD8⁺ T cells isolated from the second group of IFN-γ knockout mice were adoptively transferred into C57BL/6 (i.e., wild-type mice) that had been infected with influenza A virus H5N1 for 6 days. For these recipient mice, mortality was monitored daily until 16 dpi (i.e., IFN-γ KO CD8 T cells in FIG. 9C).

As shown in FIG. 9C (and observed in FIG. 9B), 50% of the infected C57BL/6 mice, which received CD8⁺ T cells from rIL-17A pre-treated, infected C57BL/6 mice, survived at 16 dpi (i.e., WT CD8 T cells + rIL-17A in FIG. 9C). This data was agreement with the observation described above and shown in FIG. 9B, and thus, served as a positive control for the adoptive transfer experiment of FIG. 9C. The data in FIG. 9C are representative of three independent experiments.

The infected C57BL/6 mice, which received CD8⁺ T cells from the second group of IFN-γ knockout mice (i.e., IFN-γ knockout mice not receiving rIL-17A treatment before viral challenge), died at 11 dpi (FIG. 9C, IFN-γ KO CD8 T cells). This data indicated that CD8⁺ T cells, which are unable to produce IFN-γ and were derived from mice that did not receive rIL-17 pre-treatment, were not able to protect against influenza A H5N1 viral infection. The infected C57BL/6 mice, which received CD8⁺ T cells from the first group of IFN-γ knockout mice (i.e., IFN-γ knockout mice pre-treated with rIL-17A before viral challenge), died at 12 dpi (FIG. 9C, IFN-γ KO CD8 T cells + rIL-17A). This data indicated that CD8⁺T cells, which are unable to produce IFN-γ and were derived from mice pre-treated with rIL-17A, were not able to protect against influenza A HSN1 viral infection.

In summary, the data above indicated that H5N1 infected mice recovered and survived after receiving IFN-γ producing CD8⁺ T cells from rIL-17A pre-treated mice. H5N1 infected mice that received CD8⁺ T cells unable to produce IFN-γ did not recover from and survive viral infection (regardless of rIL-17A pre-treatment). Accordingly, IFN-γ producing CD8⁺ T cells, but not those CD8⁺ T cells unable to produce IFN-γ, facilitated IL-17A-mediated protection against influenza A viral infection. CD8⁺ T cells and the IFN-γ produced from these CD8⁺ T cells, significantly contributed to IL-17A-mediated protection against influenza A viral infection.

### Example 9

### Lytic Activity of CD8⁺ T cells

CD8⁺ T cells that are cytolytic are also known as cytolytic T lymphocytes (CTLs). CTLs can have anti-viral activity. Accordingly, the IFN-γ producing CD8⁺ T cells that facilitated IL-17A-mediated protection against influenza infection were examined for cytolytic activity. In particular, in vivo and in vitro assays for cytolytic activity were used as described below.

With regards to the in vivo assay for cytolytic activity, syngeneic splenocytes were prepared from naive C57BL/6 mice (i.e., mice not receiving rIL-17A pre-treatment nor challenged with influenza virus). The syngeneic splenocytes were divided into two groups. The first group of syngeneic splenocytes was pulsed with inactivated influenza A virus H5N1 and labeled with 10 µM of carboxyfluorescein succinimidyl ester (CFSE, a fluorescent dye for staining cells). The second group of syngeneic splenocytes was labeled with 0.5 µM of CFSE. Equal numbers of splenocytes from the two groups were mixed together.

Wild-type (i.e., C57BL/6) mice and mice lacking CD8⁺ T cells (i.e., CD8 knockout mice) were pre-treated i.p. with 0.5 µg of rIL-17A on day 7 and day 2 before viral infection. A second group of C57BL/6 mice and a second group of CD8 knockout mice did not receive rIL-17A pre-treatment. The four groups of mice were challenged with 5 LD₅₀ of influenza A virus H5N1 on day 0. The H5N1 virus was administered nasally to the mice. At 6 dpi, the mixed splenocytes described above were injected intravenously into these four groups of mice. Eight hours later, splenocytes were isolated from the recipient mice to determine the level of antigen-specific cytolysis in vivo.

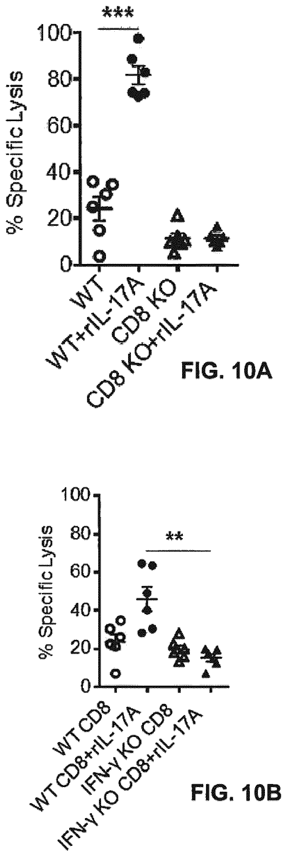

As shown in FIG. 10A, antigen-specific lysis was significantly increased in wild-type mice pre-treated with rIL-17A as compared to wild-type mice that did not receive rIL-17A pre-treatment (i.e., WT + rIL-17A and WT, respectively, in FIG. 10A). The data in FIG. 10A are representative of three independent experiments, mean ± SEM, and *** p < 0.001 (unpaired student's t-test). Specifically, antigen-specific lysis in rIL-17A pre-treated, wild-type mice was about 4-fold higher than antigen-specific lysis in wild-type mice that did not receive rIL-17A pre-treatment. This data indicated that rIL-17A pre-treatment significantly induced CTL activity.

Additionally, CD8 knockout mice receiving and not receiving rIL-17A pre-treatment had similar levels of antigen-specific cytolytic activity (FIG. 10A, CD8 KO + rIL-17A and CD8 KO, respectively). Rather, CD8 knockout mice, regardless of rIL-17A pre-treatment, had levels of antigen-specific lysis that were lower than the levels of antigen-specific lysis observed for wild-type mice. Accordingly, CTL activity was decreased about 2-fold in both CD8 knockout mice receiving and not receiving rIL-17A pre-treatment as compared to the CTL activity of wild-type mice. In summary, these data indicated that CD8⁺ T cells were required for cytolytic activity and this cytolytic activity was significantly increased in response to rIL-17A pre-treatment.

To further examine the cytolytic activity of the CD8⁺ T cells and to determine if IFN-γ production was needed for cytolytic activity, an in vitro assay was used to assess the cytolytic activity of different populations of CD8⁺ T cells. In particular, wild-type and IFN-γ knockout mice were pre-treated i.p. with 0.5 µg of rIL-17A on day 7 and day 2 before viral infection. A second group of wild-type mice and a second group of IFN-γ knockout mice did not receive rIL-17A pre-treatment. The four groups of mice were challenged with 5 LD₅₀ of influenza A virus H5N1 on day 0. The virus was administered nasally to the mice. At 6 dpi, CD8⁺ T cells were isolated from the four groups of mice and used as effector T cells.

Syngeneic splenocytes were prepared from naive C57BL/6 mice (i.e., mice not receiving rIL-17A pre-treatment nor challenged with influenza virus). The syngeneic splenocytes were divided into two groups. The first group of syngeneic splenocytes was pulsed with inactivated influenza A virus H5N1 and labeled with 10 µM of CFSE. The second group of syngeneic splenocytes was labeled with 0.5 µM of CFSE and served as a negative control.

The first and second groups of splenocytes were then independently mixed with the effector T cells isolated from each of the four groups of mice. After 3 days, specific lysis was analyzed by flow cytometry.

As shown in FIG. 10B, CD8⁺ T cells isolated from wild-type mice pre-treated with rIL-17A had higher levels of antigen-specific lysis as compared to the CD8⁺ T cells isolated from wild-type mice that did not receive rIL-17A pre-treatment (WT CD8 + rIL-17A and WT CD8, respectively, in FIG. 10B). The data in FIG. 10B are representative of three independent experiments, mean ± SEM, and **p < 0.01 (unpaired student's t-test). This data indicated that rIL-17A pre-treatment increased the cytolytic activity of CD8⁺ T cells as was also observed in the in vivo assay discussed above (FIGS. 10A and 10B). CD8⁺ T cells incapable of producing IFN-γ had significantly reduced levels of antigen-specific lytic activity as compared to CD8⁺ T cells isolated from rIL-17A pre-treated, wild-type mice (FIG. 10B, IFN-γ KO CD8 and WT CD8 + rIL-17A, respectively). Additionally, CD8⁺ T cells obtained from rIL-17A pre-treated, IFN-γ knockout mice had similar levels of antigen-specific lytic activity as the CD8⁺ T cells isolated from IFN-γ knockout mice that did not receive rIL-17A pre-treatment (FIG. 10B, IFN-γ KO CD8 + rIL-17A and IFN-γ KO CD8, respectively). CTL activity of the CD8⁺ T cells isolated IFN-γ knockout mice that did and did not receive rIL-17A pre-treatment was reduced about 3-fold and about 2-fold, respectively, as compared to the CTL activity of CD8⁺ T cells isolated from rIL-17A pre-treated, wild-type mice. These data indicated that the cytolytic activity of CD8⁺ T cells was significantly impaired in the IFN-γ knockout mice as compared to wild-type mice. These data also indicated that without IFN-γ production, the cytolytic activity of CD8⁺ T cells was not significantly induced by rIL-17A pre-treatment.

In summary, the data obtained from the in vivo and in vitro antigen-specific lysis assays demonstrated that the cytolytic activity of CD8⁺ T cells induced by rIL-17A pre-treatment was dependent upon IFN-γ production.

Accordingly, these data, along with the data from the above Examples, demonstrated that IL-17A serum levels significantly increased in both mice and humans infected with influenza virus, the severity of influenza viral infection corresponded with IL-17A serum levels in humans, IL-17A knockout mice were more susceptible to influenza viral infection, and pre-treatment with rIL-17A protected mice from death in a dose dependent manner. IL-17A-mediated protection against (and resistance to) influenza viral infection was facilitated by IFN-γ producing CD8⁺ T cells that had antigen-specific cytolytic activity. These IFN-γ producing cytotoxic CD8⁺ T cells were induced by IL-17A treatment. In summary, IL-17A treatment significantly induced an anti-viral immune response that protected the subject receiving such IL-17A treatment from influenza viral infection.

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the spirit and scope thereof.

ÐòÁÐ±í

<110>  Wang, BinµÈ

<120>  ÃâÒßÖÎÁÆ×é�ºÏÎï¡¢ÖÎÁÆ·½·¨ºÍÕï¶Ï·½·¨


<130>  VGX00134

<160>  14

<170>  PatentIn 3.5°æ

<210>  1
<211>  1115
<212>  DNA
<213>  Ð¡¼ÒÊó

<400>  1
gaggctcaag tgcacccagc accagctgat caggacgcgc aaacatgagt ccagggagag      60

cttcatctgt gtctctgatg ctgttgctgc tgctgagcct ggcggctaca gtgaaggcag     120

cagcgatcat ccctcaaagc tcagcgtgtc caaacactga ggccaaggac ttcctccaga     180

atgtgaaggt caacctcaaa gtctttaact cccttggcgc aaaagtgagc tccagaaggc     240

cctcagacta cctcaaccgt tccacgtcac cctggactct ccaccgcaat gaagaccctg     300

atagatatcc ctctgtgatc tgggaagctc agtgccgcca ccagcgctgt gtcaatgcgg     360

agggaaagct ggaccaccac atgaattctg ttctcatcca gcaagagatc ctggtcctga     420

agagggagcc tgagagctgc cccttcactt tcagggtcga gaagatgctg gtgggtgtgg     480

gctgcacctg cgtggcctcg attgtccgcc aggcagccta aacagagacc cgcggctgac     540

ccctaagaaa cccccacgtt tctcagcaaa cttacttgca tttttaaaac agttcgtgct     600

attgattttc agcaaggaat gtggattcag aggcagattc agaattgtct gccctccaca     660

atgaaaagaa ggtgtaaagg ggtcccaaac tgcttcgtgt ttgtttttct gtggacttta     720

aattatttgt gtatttacaa tatcccaaga taactttgaa ggcgtaactt atttaatgaa     780

gtatctacat tattattatg tttctttctg aagaagacaa aattcaagac tcagaaattt     840

tattatttaa aaggtaagcc tatatttata tgagctattt atgaatctat ttatttttct     900

tcagtatttg aagtattaag aacatgattt tcagatctac ctagggaagt cctaagtaag     960

attaaatatt aatggaaatt tcagctttac tatttggttg atttaaggtt ctctcctctg    1020

aatggggtga aaaccaaact tagttttatg tttaataact ttttaaatta ttgaagattc    1080

aaaaaattgg ataatttagc tccctactct gtttt                               1115


<210>  2
<211>  477
<212>  DNA
<213>  Ð¡¼ÒÊó

<400> 2
atgagtccag ggagagcttc atctgtgtct ctgatgctgt tgctgctgct gagcctggcg 60

gctacagtga aggcagcagc gatcatccct caaagctcag cgtgtccaaa cactgaggcc 120

aaggacttcc tccagaatgt gaaggtcaac ctcaaagtct ttaactccct tggcgcaaaa 180

gtgagctcca gaaggccctc agactacctc aaccgttcca cgtcaccctg gactctccac 240

cgcaatgaag accctgatag atatccctct gtgatctggg aagctcagtg ccgccaccag 300

cgctgtgtca atgcggaggg aaagctggac caccacatga attctgttct catccagcaa 360

gagatcctgg tcctgaagag ggagcctgag agctgcccct tcactttcag ggtcgagaag 420

atgctggtgg gtgtgggctg cacctgcgtg gcctcgattg tccgccaggc agcctaa 477

<210> 3
<211> 158
<212> PRT
<213> Ð¡¼ÒÊó

<400> 3

Met Ser Pro Gly Arg Ala Ser Ser Val Ser Leu Met Leu Leu Leu Leu
1               5                   10                  15

Leu Ser Leu Ala Ala Thr Val Lys Ala Ala Ala Ile Ile Pro Gln Ser
            20                  25                  30

Ser Ala Cys Pro Asn Thr Glu Ala Lys Asp Phe Leu Gln Asn Val Lys
        35                  40                  45

Val Asn Leu Lys Val Phe Asn Ser Leu Gly Ala Lys Val Ser Ser Arg
    50                  55                  60

Arg Pro Ser Asp Tyr Leu Asn Arg Ser Thr Ser Pro Trp Thr Leu His
65                  70                  75                  80

Arg Asn Glu Asp Pro Asp Arg Tyr Pro Ser Val Ile Trp Glu Ala Gln
                85                  90                  95

Cys Arg His Gln Arg Cys Val Asn Ala Glu Gly Lys Leu Asp His His
            100                 105                 110

Met Asn Ser Val Leu Ile Gln Gln Glu Ile Leu Val Leu Lys Arg Glu
        115                 120                 125

Pro Glu Ser Cys Pro Phe Thr Phe Arg Val Glu Lys Met Leu Val Gly
    130                 135                 140

Val Gly Cys Thr Cys Val Ala Ser Ile Val Arg Gln Ala Ala
145                 150                 155

<210>  4
<211>  1874
<212>  DNA
<213>  ÖÇÈË

<400>  4
gaattccggc aggcacaaac tcatccatcc ccagttgatt ggaagaaaca acgatgactc      60

ctgggaagac ctcattggtg tcactgctac tgctgctgag cctggaggcc atagtgaagg     120

caggaatcac aatcccacga aatccaggat gcccaaattc tgaggacaag aacttccccc     180

ggactgtgat ggtcaacctg aacatccata accggaatac caataccaat cccaaaaggt     240

cctcagatta ctacaaccga tccacctcac cttggaatct ccaccgcaat gaggaccctg     300

agagatatcc ctctgtgatc tgggaggcaa agtgccgcca cttgggctgc atcaacgctg     360

atgggaacgt ggactaccac atgaactctg tccccatcca gcaagagatc ctggtcctgc     420

gcagggagcc tccacactgc cccaactcct tccggctgga gaagatactg gtgtccgtgg     480

gctgcacctg tgtcaccccg attgtccacc atgtggccta agagctctgg ggagcccaca     540

ctccccaaag cagttagact atggagagcc gacccagccc ctcaggaacc ctcatccttc     600

aaagacagcc tcatttcgga ctaaactcat tagagttctt aaggcagttt gtccaattaa     660

agcttcagag gtaacacttg gccaagatat gagatctgaa ttacctttcc ctctttccaa     720

gaaggaaggt ttgactgagt accaatttgc ttcttgttta cttttttaag ggctttaagt     780

tatttatgta tttaatatgc cctgagataa ctttggggta taagattcca ttttaatgaa     840

ttacctactt tattttgttt gtctttttaa agaagataag attctgggct tgggaatttt     900

attatttaaa aggtaaaacc tgtatttatt tgagctattt aaggatctat ttatgtttaa     960

gtatttagaa aaaggtgaaa aagcactatt atcagttctg cctaggtaaa tgtaagatag    1020

aattaaatgg cagtgcaaaa tttctgagtc tttacaacat acggatatag tatttcctcc    1080

tctttgtttt taaaagttat aacatggctg aaaagaaaga ttaaacctac tttcatatgt    1140

attaatttaa attttgcaat ttgttgaggt tttacaagag atacagcaag tctaactctc    1200

tgttccatta aacccttata ataaaatcct tctgtaataa taaagtttca aaagaaaatg    1260

tttatttgtt ctcattaaat gtattttagc aaactcagct cttccctatt gggaagagtt    1320

atgcaaattc tcctataagc aaaacaaagc atgtctttga gtaacaatga cctggaaata    1380

cccaaaattc caagttctcg atttcacatg ccttcaagac tgaacaccga ctaaggtttt    1440

catactatta gccaatgctg tagacagaag cattttgata ggaatagagc aaataagata    1500

atggccctga ggaatggcat gtcattatta aagatcatat ggggaaaatg aaaccctccc    1560

caaaatacaa gaagttctgg gaggagacat tgtcttcaga ctacaatgtc cagtttctcc    1620

cctagactca ggcttccttt ggagattaag gcccctcaga gatcaacaga ccaacatttt    1680

tctcttcctc aagcaacact cctagggcct ggcttctgtc tgatcaaggc accacacaac 1740

ccagaaagga gctgatgggg cagaatgaac tttaagtatg agaaaagttc agcccaagta 1800

aaataaaaac tcaatcacat tcaattccag agtagtttca agtttcacat cgtaaccatt 1860

ttcgcccgga attc 1874

<210> 5
<211> 468
<212> DNA
<213> ÖÇÈË

<400> 5
atgactcctg ggaagacctc attggtgtca ctgctactgc tgctgagcct ggaggccata 60

gtgaaggcag gaatcacaat cccacgaaat ccaggatgcc caaattctga ggacaagaac 120

ttcccccgga ctgtgatggt caacctgaac atccataacc ggaataccaa taccaatccc 180

aaaaggtcct cagattacta caaccgatcc acctcacctt ggaatctcca ccgcaatgag 240

gaccctgaga gatatccctc tgtgatctgg gaggcaaagt gccgccactt gggctgcatc 300

aacgctgatg ggaacgtgga ctaccacatg aactctgtcc ccatccagca agagatcctg 360

gtcctgcgca gggagcctcc acactgcccc aactccttcc ggctggagaa gatactggtg 420

tccgtgggct gcacctgtgt caccccgatt gtccaccatg tggcctaa 468

<210> 6
<211> 155
<212> PRT
<213> ÖÇÈË

<400> 6

Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155

<210>  7
<211>  498
<212>  DNA
<213>  ÈË¹¤ÐòÁÐ

<220>
<223>  ÓÅ»¯µÄÐ¡ÊóIL-17A°ËÜÕËáÐòÁÐ

<400>  7
ggatccgcca ccatgtctcc aggtcgggct tccagtgtct ctctgatgct gctgctgctg      60

ctctccctcg ccgccacagt gaaggctgcc gctattattc ctcagagctc cgcctgccca     120

aacactgagg ctaaggactt cctgcagaac gtgaaggtca atctgaaagt gtttaattcc     180

ctcggcgcta aagtctctag taggagaccc tctgattacc tgaacaggtc aaccagccct     240

tggacactcc accgaaatga ggaccccgat aggtatccta gcgtgatctg ggaagcacag     300

tgccggcatc agaggtgcgt gaacgccgag ggaaagctgg accaccatat gaatagtgtg     360

ctcatccagc aggaaattct ggtcctcaaa agagagccag aatcctgtcc cttcaccttt     420

cgggtggaaa agatgctggt cggggtgggt tgtacttgcg tggcttccat tgtgagacag     480

gctgcctgat aactcgag                                                   498

<210>  8
<211>  474
<212>  DNA
<213>  ÈË¹¤ÐòÁÐ

<220>
<223>  ÓÅ»¯µÄÐ¡ÊóIL-17A±àÂëÐòÁÐ

<400>  8
atgtctccag gtcgggcttc cagtgtctct ctgatgctgc tgctgctgct ctccctcgcc      60

gccacagtga aggctgccgc tattattcct cagagctccg cctgcccaaa cactgaggct     120

aaggacttcc tgcagaacgt gaaggtcaat ctgaaagtgt ttaattccct cggcgctaaa     180

gtctctagta ggagaccctc tgattacctg aacaggtcaa ccagcccttg gacactccac     240

cgaaatgagg accccgatag gtatcctagc gtgatctggg aagcacagtg ccggcatcag     300

aggtgcgtga acgccgaggg aaagctggac caccatatga atagtgtgct catccagcag     360

gaaattctgg tcctcaaaag agagccagaa tcctgtccct tcacctttcg ggtggaaaag     420

atgctggtcg gggtgggttg tacttgcgtg gcttccattg tgagacaggc tgcc           474

<210>  9
<211>  158
<212>  PRT
<213>  ÈË¹¤ÐòÁÐ

<220>
<223>  ÓÉÓÅ»¯µÄÐ¡ÊóIL-17A�ºËËáÐòÁÐ±àÂëµÄ°±»ùËáÐòÁÐ

<400>  9

Met Ser Pro Gly Arg Ala Ser Ser Val Ser Leu Met Leu Leu Leu Leu
1               5                   10                  15

Leu Ser Leu Ala Ala Thr Val Lys Ala Ala Ala Ile Ile Pro Gln Ser
            20                  25                  30

Ser Ala Cys Pro Asn Thr Glu Ala Lys Asp Phe Leu Gln Asn Val Lys
        35                  40                  45

Val Asn Leu Lys Val Phe Asn Ser Leu Gly Ala Lys Val Ser Ser Arg
    50                  55                  60

Arg Pro Ser Asp Tyr Leu Asn Arg Ser Thr Ser Pro Trp Thr Leu His
65                  70                  75                  80

Arg Asn Glu Asp Pro Asp Arg Tyr Pro Ser Val Ile Trp Glu Ala Gln
                85                  90                  95

Cys Arg His Gln Arg Cys Val Asn Ala Glu Gly Lys Leu Asp His His
            100                 105                 110

Met Asn Ser Val Leu Ile Gln Gln Glu Ile Leu Val Leu Lys Arg Glu
        115                 120                 125

Pro Glu Ser Cys Pro Phe Thr Phe Arg Val Glu Lys Met Leu Val Gly
    130                 135                 140

Val Gly Cys Thr Cys Val Ala Ser Ile Val Arg Gln Ala Ala
145                 150                 155

<210>  10
<211>  489
<212>  DNA
<213>  ÈË¹¤ÐòÁÐ

<220>
<223>   ÓÅ»¯µÄÈËIL-17A°ËÜÕËáÐòÁÐ

<400>   10
ggatccgcca ccatgacccc tggaaaaaca agtctggtgt ctctgctgct gctgctgtct      60

ctggaggcaa tcgtcaaggc tggaatcaca atcccacgga acccaggctg ccccaattcc     120

gaggacaaga acttccctag aactgtgatg gtcaacctga atatccacaa caggaatacc     180

aacacaaatc ccaaacgaag ctccgactac tataatcggt ctaccagtcc ttggaacctg     240

cataggaatg aggaccccga acgctaccca agtgtgatct gggaagccaa gtgccgccac     300

ctgggctgta ttaacgctga cgggaatgtg gattatcata tgaattcagt cccaatccag     360

caggagattc tggtcctgcg gagagaaccc cctcactgtc ccaacagctt tcgactggaa     420

aaaatcctgg tgtccgtggg ctgtacctgc gtgactccaa tcgtccatca tgtggcttga     480

taactcgag                                                             489


<210>   11
<211>   465
<212>   DNA
<213>   ÈË¹¤ÐòÁÐ

<220>
<223>   ÓÅ»¯µÄÈËIL-17A±àÂëÐòÁÐ

<400>   11
atgacccctg gaaaaacaag tctggtgtct ctgctgctgc tgctgtctct ggaggcaatc      60

gtcaaggctg gaatcacaat cccacggaac ccaggctgcc ccaattccga ggacaagaac     120

ttccctagaa ctgtgatggt caacctgaat atccacaaca ggaataccaa cacaaatccc     180

aaacgaagct ccgactacta taatcggtct accagtcctt ggaacctgca taggaatgag     240

gaccccgaac gctacccaag tgtgatctgg gaagccaagt gccgccacct gggctgtatt     300

aacgctgacg ggaatgtgga ttatcatatg aattcagtcc caatccagca ggagattctg     360

gtcctgcgga gagaaccccc tcactgtccc aacagctttc gactggaaaa aatcctggtg     420

tccgtgggct gtacctgcgt gactccaatc gtccatcatg tggct                     465


<210>   12
<211>   155
<212>   PRT
<213>   ÈË¹¤ÐòÁÐ

<220>
<223>   ÓÉÓÅ»¯µÄÈËIL-17A°ËËáÐòÁÐ±àÂëµÄ°±»ùËáÐòÁÐ

<400>   12

Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155

<210>  13
<211>  54
<212>  DNA
<213>  ÈË¹¤ÐòÁÐ

<220>
<223>  IgEÇ°µ¼ÐòÁÐ

<400>  13
atggactgga cttggattct gttcctggtc gccgccgcaa ctcgcgtgca tagc          54

<210>  14
<211>  18
<212>  PRT
<213>  ÈË¹¤ÐòÁÐ

<220>
<223>  IgEÇ°µ¼ÐòÁÐ

<400>  14

Met Asp Trp Thr Trp Ile Leu Phe Leu Val Ala Ala Ala Thr Arg Val
1               5                   10                  15

His Ser

## Claims

1. A method for treating a subject in need thereof against influenza virus, the method comprising administering a immunotherapeutic composition comprising IL-17 to the subject, wherein the subject is thereby resistant against multiple influenza virus subtypes.

2. The method of claim 1, wherein IL-17 is a peptide comprising the amino acid sequence as set forth in SEQ ID NO: 12.

3. The method of claim 1, wherein administering the immunotherapeutic composition includes electroporation or injection.

4. The method of claim 1, wherein the influenza virus is an influenza A virus.

5. The method of claim 4, wherein the influenza A virus is subtype H5N1, H7N9, or H1N1.

6. The method of claim 5, wherein the influenza A virus is subtype H5N1.

7. The method of claim 5, wherein the influenza A virus is subtype H7N9.

8. The method of claim 1, wherein an immune response of the subject is increased by at least about 4-fold.

9. The method of claim 8, wherein the immune response of the subject is increased by at least about 2-fold.

10. The method of claim 1, wherein an increased immune response of the subject provides at least about 70% survival of influenza virus.

11. The method of claim 10, wherein the increased immune response of the subject provides at least about 100% survival of influenza virus.

12. The method of claim 2, wherein the immunotherapeutic composition further comprises a nucleic acid encoding the IL-17 peptide.

13. The method of claim 2, wherein the immunotherapeutic composition further comprises one or more other antigens selected from the group consisting of amiloride, an influenza antigen, and an interleukin.

14. A method for diagnosing influenza viral infection in a subject in need thereof, the method comprising:
(a) providing a sample obtained from the subject;
(b) measuring a level of IL-17 in the sample;
(c) comparing the measured level of IL-17 to a threshold level of IL-17; and
(d) determining the subject is infected with influenza virus if the measured level of IL-17 is higher than the threshold level of IL-17.

15. The method of claim 14, wherein IL-17 is a peptide comprising the amino acid sequence as set forth in SEQ ID NO: 12.

16. The method of claim 14, wherein the sample is a blood sample, a serum sample, or a plasma sample.

17. The method of claim 14, further comprising administering an immunotherapeutic composition comprising IL-17 to the subject infected with the influenza virus.

18. The method of claim 17, wherein IL-17 is a peptide comprising the amino acid sequence as set forth in SEQ ID NO: 12.

19. The method of claim 14, further comprising distinguishing between a mild influenza viral infection and a severe influenza viral infection.

20. The method of claim 14, wherein the influenza virus is subtype H5N1, H1N1, or H7N9.

21. A immunotherapeutic composition comprising IL-17.

22. The immunotherapeutic composition of claim 21, wherein IL-17 is encoded by SEQ ID NO:10 or SEQ ID NO:11.

23. The immunotherapeutic composition of claim 21, wherein IL-17 is a peptide comprising the amino acid sequence as set forth in SEQ ID NO: 12.

24. The immunotherapeutic composition of claim 21, wherein IL-17 is encoded by a nucleic acid, and wherein the immunotherapeutic composition further comprises an IL-17 peptide.

25. The immunotherapeutic composition of claim 21, further comprising one or more other antigens selected from the group consisting of amiloride, an influenza antigen, and an interleukin.

26. The immunotherapeutic composition of claim 25, wherein the influenza antigen is selected from the group consisting of H1 HA, H2 HA, H3 HA, H5 HA, BHA antigen, and any combination thereof.

27. The immunotherapeutic composition of claim 25, wherein the interleukin is selected from the group consisting of IL-23, IL-33, IL-21 and the combination thereof.

28. The immunotherapeutic composition of claim 21, further comprising a pharmaceutically acceptable excipient.

29. The immunotherapeutic composition of claim 21, wherein IL-17 is a monomer of an IL-17 peptide.

30. The immunotherapeutic composition of claim 21, wherein IL-17 is a dimer of IL-17 peptides.
